# EUROPEAN PATENT APPLICATION

(11) **EP 2 444 093 A1**
(43) Date of publication of application: **25.04.2012**
(21) Application number: 11175292.9
(22) Date of filing: 10.03.2006
(51) Int. Cl.: A61K 31/70, A61K 31/353, A61K 31/365, A61K 31/366, A61K 31/415, A61K 36/185, A61K 36/47, A61K 36/28, A61K 36/48, A61K 36/15, A61K 36/54, A61K 36/53, A61K 36/60, A61K 36/906

(54) **Formulation of a mixture of free-b-ring flavonoids and flavans as a therapeutic agent**

(30) Priority: 10.03.2005 US 660564 P
(62) Divisional of application: 06737881.0
(71) Applicant: Unigen, Inc., Lacey, WA 98516 (US)
(72) Inventor: Jia, Qi, Olympia, WA Washington 98502 (US); Zhao, Yuan, Olympia, WA Washington 98502 (US)
(74) Representative: Friedrich, Rainer

(57) **Abstract**

The present invention provides a composition of matter comprised of a mixture of two specific classes of compounds - - Free-B-Ring flavonoids and flavans - referred to herein as UP736 for use in the prevention and treatment of diseases and conditions related to platelet aggregation and platelet-induced thrombosis. The invention further provides a novel composition of matter comprised of UP736 in combination with injectable or oral anticoagulants, antiplatelet agents, non-steroidal anti-inflammatory drugs (NSAIDs) and COX-2 selective inhibitors and a method for using said composition in the prevention and treatment of diseases and conditions related to platelet aggregation and platelet-induced and platelet-induced thrombosis. Finally, this invention provides a method for using UP736 in combination with anti-platelet, anti-coagulant, prophylaxis agents and NSAIDs as a means for reducing the dosage of these agents, decreasing the side effects associated with acute or chronic administration of these agents; counteracting or antagonizing the risks of acute or chronic administration of these agents and for achieving additional and/or multiple clinical benefits.

## Description

### FIELD OF THE INVENTION

This invention relates to the prevention and treatment of diseases and conditions related to platelet aggregation and platelet-induced thrombosis. Specifically, the present invention relates to a novel composition of matter comprised of a mixture of a blend of two specific classes of compounds --Free-B-Ring flavonoids and flavans-- also referred to herein as UP736 for use in the prevention and treatment of diseases and conditions mediated by platelet aggregation and platelet-induced thrombosis. This invention further relates to a method for using UP736 as an adjuvant and/or a synergistic, and/or a potentiating agent in conjunction with injectable or oral anticoagulants, antiplatelet agents, non-steroidal anti-inflammatory drugs (NSAIDs) and COX-2 selective inhibitors. Finally, this invention relates to a method for using UP736 in combination with anti-platelet, anti-coagulant, prophylaxis agents and NSAIDs as a means for reducing the dosage of these agents, decreasing the side effects associated with acute or chronic administration of these agents; counteracting or antagonizing the risks of acute or chronic administration of these agents and for achieving additional and/or multiple clinical benefits.

### BACKGROUND OF THE INVENTION

The liberation and metabolism of arachidonic acid (AA) from the cell membrane results in the generation ofmetabolites by several different pathways. Arguably, two of the most important pathways are mediated by the enzymes 5-lipoxygenase (LOX) and cyclooxygenase (COX). These are parallel pathways result in the generation of leukotrienes and prostaglandins, respectively, which play important roles in the initiation and progression of the inflammatory response and platelet aggregation. Consequently, the enzymes responsible for generating these mediators have become the targets for many new drugs aimed at the treatment of inflammation and modulation of platelet aggregation that contributes to the pathogenesis of diseases such as rheumatoid arthritis, osteoarthritis, and atherothrombosis.

Inhibition of the COX enzyme is the mechanism of action attributed to most non-steroidal anti-inflammatory drugs (NSAIDS). There are two distinct isoforms of the COX enzyme (COX-1 and COX-2), which share approximately 60% sequence homology, but differ in expression profiles and function. COX-1 is a constitutive form of the enzyme that has been linked to the production ofphysiologically important prostaglandins, which help regulate normal physiological functions, such as platelet aggregation, protection of cell function in the stomach and maintenance of normal kidney function. (Dannhardt and Kiefer (2001) Eur. J. Med. Chem. 36:109-26). The second isoform, COX-2, is a form of the enzyme that is inducible by pro-inflammatory cytokines, such as interleukin-1β (IL-1β) and other growth factors. (Herschmann (1994) Cancer Metastasis Rev. 134:241-56; Xie et al. (1992) Drugs Dev. Res. 25:249-65). This isoform catalyzes the production of prostaglandin E₂ (PGE2) from arachidonic acid (AA). Because the mechanism of action of COX inhibitors overlaps that of most conventional NSAIDs, COX inhibitors are used to treat many of the same symptoms, including atherothrombosis, pain and swelling associated with inflammation in transient conditions and chronic diseases.

Platelets play a central role in normal hemostasis. After vascular injury, platelets leak out into the extracellular matrix through the damaged endothelial wall, where they are activated by various constituents in the extracellular matrix including collagen, proteoglycans, fibronectin and other adhesive glycoproteins. On contact with the extracellular matrix, platelets undergo multiple and sequential reactions including adhesion and shape change, secretion of two types of granules and aggregation. Two potent platelet aggregation mediators: adenine diphosphate (ADP) and thromboxane A2 (TxA2) have been identified. ADP is released from platelets after they are activated by extracellular matrix constituents. In addition to mediating aggregation ofplatelets, ADP also enhances ADP release from other platelets forming a positive feedback loop for platelet aggregation. TxA2 is synthesized and released from platelets, and is an important stimulus for platelet aggregation as well. Together with ADP, TxA2 sets up an autocatalytic reaction leading to build-up of an enlarging platelet aggregate. Aggregated platelets are important for the subsequent blood coagulation process. These activated platelets stimulate local activation of plasma coagulation factors, leading to generation of a fibrin clot that reinforces the platelet aggregate. Recent studies suggest that all of the membrane-bound reactions of the coagulation system can be localized to the surface of activated platelets (Conde *et al.* (2005) Blood 106:1604-1611).

Although the adhesion and activation of platelets is a repair-oriented response to sudden vascular injury, uncontrolled progression this process through a series of self-sustaining amplification loops may lead to the intraluminal formation of thrombus, vascular occlusion, and transient ischemia or infarction (Ruggeri (2002) Nat. Med. 8:1227-34). The ability ofplatelets to participate in both normal hemostasis and atherothrombosis depends on their adhesive properties and their capacity to become activated very quickly in response to various stimuli.

Natural platelets express only COX-1. Platelets process PGH2 to produce primarily TxA2, which is synthesized and released by platelets in response to collagen, thrombin and other stimuli. TxA2 induces irreversible platelet aggregation through its interaction with a G-protein―coupled receptor, the TxA2 receptor. Thus, TxA2 provides a mechanism for amplifying the responses ofplatelets to diverse agonists. In addition, TxA2 is a potent vasoconstrictor, induces the proliferation ofvascular smooth-muscle cells, and is proatherogenic. As a vasoconstrictor TxA2 promotes proper platelet aggregation. By inhibiting the COX-1 enzyme, aspirin will reduce the production ofTxA2 which leads to reduced platelet aggregation (Patrono et al. (2006) The New England Journal Medicine. 353:22: 237).

Prostacyclins are produced in the endothelial lining of arteries and the heart. The balance between prostacyclin (PGI₂), a strong vasodilator, and that ofthromboxanes, such as TxA2, is crucial to maintaining proper cardiovascular function (Bunting et al. (1983) Br. Med. Bull. 39:271). Both PGI₂ and TxA2 are dependent upon the production of COX-1 and COX-2 in the endothelial lining of arteries and in the cardio tissue of the heart (Caughey et al. (2001) J Immunol, 167:2831; Ribuot et al. (2003) Cardiovascular Res 58:582). COX-1 and COX-2 ratios have been shown to affect the balance of both PGI₂ and TxA2. COX-1 metabolizes arachidonic acid converting the fatty acid primarily to TxA2, whereas induced COX-2 processes arachidonic acid transforming it to PGE₂ and PGI₂ (Oh-ishi (1997) Biochem. Biophys. Res. Commun. 230:110; Brock et al. (1999) J. Biol. Chem. 274:11660). PGI2 inhibits platelet aggregation in response to all agonists through its interaction with the PGI2 receptor. TxA2 is a prostanoid largely derived from COX-1 (mostly from platelets) and its biosynthesis is highly sensitive to inhibition by aspirin (Rocca et al. (2002) Proc. Natl. Acad. Sci. USA 99:7634-9). PGI2, on the other hand, is derived predominantly from COX-2 (McAdam et al. (1999) Proc. Natl. Acad. Sci. USA 96:272-7) and is less susceptible to inhibition by aspirin. Highly selective inhibition of COX-2 may promote thrombosis by tipping the balance of the synthesis of PGI₂ (COX-2 pathway) over TxA2 (COX-1 pathway) via the shunting of arachidonic acid within the eicosanoid COX-1 pathway. (Gaetano (2003) Trends in Pharmacological Sciences. 24(5):245-252).

Platelet aggregation plays a very important role in the inducement and development of athrothrombosis, which is the major cause of deep vein thrombosis, pulmonary embolism, atherosclerosis, myocardial infarction, thrombosis in cerebral vessels and/or embolism of cerebral vessels leading to cerebrovascular events. Antiplatelet drugs, such as aspirin, and anticoagulation drugs, such as heparin and warfarin (Verheugt (2005) Presse Med. 34:1325), thrombin specific inhibitors, such as hirudin, desirudin, bivalirudin and thrombin non-specific inhibitors, such as statins (Shen (2006) Front Biosci. 11:113) are currently the standard drugs used to manage of thromboemboliam. However, complications arising from serious bleeding are a major side effect of anticoagulation drugs and from high dose short-term antiplatelet therapy. The use of smaller doses of anticoagulation drugs combined with moderate to low doses of antiplatelet compounds, such as aspirin has been shown to have a significant therapeutic value in the reducing the threat of bleeding in high-risk patients (Harrington et al. (2004) Chest. 126.3 Suppl.513S).

Due to the irreversible inhibition ofplatelet cyclooxygenase and the prevention of the formation of TxA2, aspirin type drugs have also been utilized over the long term for reducing the risks of cardiovascular disease, in preventing acute myocardial infarction and in preventing acute occlusive stroke (Hennekens (2002) Am. J. Manag. Care 8(22 Suppl.):S691). The most common side effect resulting from long term use of aspirin and other anti-platelet salicylates is local erosion of the gastric mucosa due to the inhibition of COX-1, which is important in maintaining the integrity of the mucosa lining. Such damage of the gastric mucosa can lead from occult blood loss to acute GI hemorrhage due to serious gastoduodental injury. Short-term high dose administration of anti-platelet drugs also has its own risks, such as significantly increased stroke potential and bleeding after surgical procedures. The adjustment of optimum dosage is one option for reducing these side effects (Kong (2004) Am. J. Cardiovasc. Drugs 4(3):151). Daily doses ranging from 75 mg-150 mg are recommended for long-term preventive use. Certainly any compounds that can improve aspirins antiplatelet effect without increasing its side effects will have significant therapeutic advantages (Patrono et al. (2005) New Eng. J. Med. 353:22; 2373). Unfortunately, there is currently no such option available, though the use of an antisecretory agents, such as a proton pump inhibitor can reduce the risk of upper gastrointestinal bleeding in patients taking anti-platelet drugs.

In order to address aspirin and other classical NSAID toxicity, particularly gastrointestinal ulceration and hemorrhage resulting from selective COX-1 inhibition, two strategies have been implemented in the drug discovery process. The first strategy involves searching for selective inhibitors of COX-2, which reduce gastrointestinal side effects by sparing COX-1 protective functions in gastric mucosa (DeWitt (1999) Mol. Pharmac. 4:625-631). This effort has lead to the successful launch of several commercial drugs, such as Celecoxib and Rofecoxib, which exhibit selectivity against COX-2. In clinical trials, COX-2 selective inhibitors demonstrated significant potency against pain and other symptoms of inflammation with lower incidence of gastrointestinal events. However, a number of side effects associated with the use of selective COX-2 inhibitors have gradually emerged. For example, these compounds have been found to promote allergic and asthmatic attacks, cause acute renal failure, congestive heart failure, exacerbate coronary and cerebrovascular diseases, delay broken bone growth and healing of ulcers, suppress the immune system making one susceptible to viral meningitis attack, and promote the development of ulcers in patients with gastric erosions or with *Helicobactor pylori* infection (Rainsford (2001) J. Physiol. - Paris 95:11-19). Recent reports that a significant anti-inflammatory effect for some highly selective COX-2 inhibitors was only observed after the dosage level reached levels in which COX-1 activity was also inhibited (Wallace et al.91999) Br. J. Pharmac. 126:1200-1204.), together with anti-inflammatory prostanoid generation by the COX-2 enzyme at a later phase of the inflammation process (Gilroy et al.(1999) Nature Med. 5:698-701), has further challenged the efficacy of selective COX-2 inhibitors.

In 2004, the drug Vioxx (Rofecoxib) was voluntarily withdrawn from the market after a clinical trial showed that over time this highly selective COX-2 inhibitor increased the risk of heart attack by greater than two-fold compared to another NSAID, Naproxen. Additionally, a clinical trial involving Celebrex (Celecoxib), sponsored by the National Cancer Institute revealed that a long-term high dose use of this COX-2 selective inhibitor more than doubled the risk of heart attack. Concerns of cardiovascular risk from another selective COX-2 inhibitor, Bextra (Valdecoxib), have also been raised (Meier B. Marketing Intensified Trouble for Pain Pills. The New York Times, December 19, 2004). In fact, a review of the recent scientific literature reveals that the increased risk of a cardiovascular event from Rofecoxib (Vioxx) and other selective COX-2 inhibitors were observed as early as the year 2000 (Juni et al. (2004) Lancet. 364(9450):2021-2029; Clark (2004) Drug Safety 27(7):427-456). There is increased evidence, which indicates that a primary cause of this cardiac toxicity is the extremely high COX-2 selectivity of this class of drugs. (Neal et al. (2004) J. Pharm. Sci. 7(3):332-336).

Recent data indicates that COX-2 is expressed in healthy organs, such as the kidneys macula densa/cTALH and medullary interstitial cells (Harris et al. (Aug. 2004) Acta Physiol Scand. 181(4):543-7); in endothelial cells (Parente and Perretti (Jan. 2003) Biochem Pharmacol. 65(2):153-9.); and in the brain (Hoffmann (Nov. 2000) Curr Med Chem. 7(11):1113-20). In the kidneys, the COX-2 enzyme is required for the production of PGE₂ and PGI₂ (prostacyclin) from arachidonic acid PGI₂, in particular, is a key regulator of sodium balance in the body (Harris (2000) J Am Soc Nephrol 11:2387). Inhibition of PGE₂ and PGI₂ by COX-2 selective inhibitors within the kidneys leads to sodium and water retention and elevation of blood pressure, as PGE₂ decreases sodium reabsorption, whereas PGI₂ is a strong vasodilator which maintains the balance between renal blood flow and glomerular filtration rate, or in simpler terms, the amount of urine produced in the body. PGI₂ also stimulates renin release, which causes an increase in the release of aldosterone, which then increases sodium reabsorption and potassium secretion. (Carmichael and Shankel (1985) Am J Med 78:992; Whelton and Hamilton (1991) J Clin Pharmacol 31:588). To maintain the proper renal perfusion, the kidneys up-regulate the synthesis of PGI₂ to counteract the effects of vasoconstrictors to maintain proper kidney function. Most healthy individuals maintain proper blood pressure on their own balancing the intake of fluids with the excretion of urine without interference from compounds causing vasoconstriction or vasodilation. In these individuals, the effects of vasoconstrictors counterbalanced by PGI₂ are not needed But in those individuals with high blood pressure, Vioxx was found to further increase bloodpressure (Lamarque (2004) Bulletin du Cancer (Montrouge) 91:S117; Whelton et al. (2001) Am J Ther 85:85). This increase in blood pressure may contribute to the increased incidence of acute myocardial infarction (AMI) (Deray (2004) Presse Med 33:483).

COX-2 enzymes also induce the expression of PGE₂ and PGI₂ in the heart, which protect against acute myocardial infarction (AMI) (Dai and Kloner (2004) J Cardiovascul Pharmacol Therapeutics 9:51). Recent studies in both rabbits and mice have shown that during an induced AMI, COX-2 is significantly up-regulated acting to stunt the event as an anti-infarct mediator (Shinmura et al. (2000) PNAS 97:10197; Guo et al. (2000)Basic Res Cardiol 95:479). This anti-infarct activity prevents further damage from occurring thereby preserving cardio function. (Bolli et al. (2002) Am J Physiol 282:H1943). In animal models, researchers have shown that PGI₂ levels were abolished when rats were administered a selective COX-2 inhibitor versus a placebo. This lack of PGI₂ prevented the rat's hearts from counteracting an induced AMI event (Bolli et al. (2002) Am J Physiol 282:H1943; Shinmura et al. (2002) Am. J Physiol 283:H2534). When COX-2 is selectively inhibited, TxA2 is produced at a much higher level in comparison to PGI₂. Vasoconstriction by TxA2 is counterbalanced by PGI₂-induced vasodilatation, which reduces blood flow in the arteries around the heart. This reduction in blood flow and limitation of nutrients and oxygen delivery may tip the balance in susceptible patients toward AMI (Bing and Lomnicka (2002) J. Am. Coll. Cardiol 39:521).

In summary, the recent evaluation of cyclooxygenase isoforms and their function have demonstrated that the lack of appreciable COX-1 inhibition is a plausible explanation for the observed increase in cardiovascular side effects associated with Vioxx (Rofecoxib) and other highly selective COX-2 inhibitors. There is even a recommendation that the use of highly COX-2 selective NSAIDs without the use of suitable COX-1 inhibitors (e.g., low dose aspirin) should avoided. (Neal et al. (2004) J. Pharm. Pharmaceut. Sci. 7(3):332-336).

Recent anti-inflammatory efforts have focused on searching for agents, which inhibit both cyclooxygenase and lipoxygenase (Parente (2001) J. Rheumatol. 28:2375-2382; Bertolini et al. (2001) Pharmac. Res. 44:437-450). Inhibitors that demonstrate dual specificity for COX and LOX would have the obvious benefit of inhibiting multiple pathways of arachidonic acid metabolism. Such inhibitors would block the inflammatory effects of prostaglandins (PG), as well as, those of multiple leukotrienes (LT) by limiting their production. This includes the vasodilation, vasopermeability and chemotactic effects of PGE2, LTB4, LTD4 and LTE4, also known as the slow reacting substance of anaphalaxis. Of these, LTB4 has the most potent chemotactic and chemokinetic effects. (Moore (1985) in Prostanoids: pharmacological, physiological and clinical relevance, Cambridge University Press, N.Y., pp. 229-230).

The significance ofblocking the inflammatory effects of PGE₂, as well as, those of multiple leukotrienes (LT) was based on the recent discovery that the significant drawbacks of selective COX-2 inhibitors are associated with the shunting of the arachidonic acid pathway to the lipoxygenase pathway, thereby causing the overproduction of pro-inflammatory, chemotactic, gastro-damaging, and bronchoconstrictive leukotrienes (Celotti and Laufer (2001) Pharmac. Res. 43:429-436).

It has been determined that NSAID induced gastric inflammation is largely due to metabolites of LOX, particularly LTC4 and LTB4 (Kirchner et al. (1997) Prostaglandins Leukot. Essent. Fatty Acids 56:417-423). Leukotrienes contribute to a significant amount of the gastric epithelial injury by stimulating leukocyte infiltration, occluding microvessels, reducing mucosal blood flow and releasing mediators, proteases and free radicals. Selective LOX inhibitors have demonstrated significant reduction in the severity or prevention of indomethacin-induced ulcer formation (Fosslien (1998) Annals Clin. Lab. Sci 28:67-81). It has also been determined that by inhibiting COX pathways, aspirin and other COX inhibitors divert arachidonic acid metabolites to the LOX pathway causing increased bronchoconstrictive leukotriene release along with an increase in the levels of cysteinyl leukotrienes, which leads to chronic rhinoconjunctivitis, nasal polyps, and asthma akin to a protracted viral respiratory infection. The prevalence of aspirin induced asthma (AIA) in the asthmatic population is about 10 to 20% and anti-leukotriene drugs have been utilized in the treatment of patients with AIA. (Babu and Salvi (2000) Chest 118:1470-1476).

Dual inhibitors also demonstrate other therapeutic benefits. They have been found to reduce coronary vasoconstriction in arthritic hearts in a rat model (Gok et al. (2000) Pharmac. 60:41-46), and significantly decrease angiotensin II-induced contractions in the human internal mammary artery (Stanke-Labesque et al. (2000) Cardiovascular Res. 47:376-383). Opioid receptor activation can cause a presynaptic inhibition of neurotransmitter release mediated by LOX metabolites of arachidonic acid in midbrain neurons. The efficacy of opioids is enhanced synergistically by treatment of brain neurons with COX and LOX dual inhibitors. This might lead to development of CNS analgesic medications involving combinations of lowered doses of opioids and COX/LOX dual inhibitors (Christie et al. (1999) Inflamm. Res. 48:1-4). COX and LOX dual inhibitors can also prevent lens protein-induced ocular inflammation in both the early and late phases (Chang et al. J. Ocular Pharmac. 5:353-360).

Dual inhibitors of COX and LOX not only suppress prostaglandins that contribute to acute inflammatory conditions, but also address the accumulation of phagocytic leukotrienes that are directly associated with chronic inflammatory symptoms. Additionally, dual inhibitors also provide cardiac protection from COX-1 inhibitory activity. These characteristics suggest that there may be distinct advantages to dual inhibitors of COX and LOX over selective COX-2 inhibitors and NSAIDs. This concept has been shown to be valid in *in vivo* models with synthetic drug candidates (Fiorucci et al. (2001) Biochem. Pharmac. 62:1433-1438).

### SUMMARY OF THE INVENTION

The present invention relates generally to a composition of matter formulated for use in the prevention and treatment of diseases and conditions related to platelet aggregation and platelet-induced thrombosis. This composition of matter is referred to herein as UP736. The composition of matter is comprised of a mixture of two specific classes of compounds --Free-B-Ring flavonoids and flavans. Compositions comprised of Free-B-Ring flavonoids, flavans and mixtures thereof are described in U.S. Application Serial No. 10/091,362, filed March 1, 2002, entitled "Identification of Free-B-Ring Flavonoids as Potent COX-2 Inhibitors," U.S. Application Serial No. 10/104,477, filed March 22, 2002, entitled "Isolation of a Dual Cox-2 and 5-Lipoxygenase Inhibitor from Acacia" and U.S. Application Serial No. 10/427,746, filed July 22, 2003, entitled "Formulation with Dual Cox-2 and 5-Lipoxygenase Inhibitory Activity." Each of these references is incorporated herein by reference in its entirety.

Included in the present invention is a novel composition of matter comprised of a mixture of at least one Free-B-Ring flavonoid, at least one flavan and at least one agent selected from the group consisting of an injectable anticoagulant, selected from the group including, but not limited to heparin, dalteparin, enoxaparin and tinzaparin; an oral anticoagulant, selected from the group including, but not limited to warfarin, vitamin K antagonists and vitamin K reductase inhibitors; an antiplatelet agent, selected from the group including, but not limited to aspirin, clodipogrel and dipyridamole; an anti-angina drug, selected from the group including, but not limited to nitrates, beta-blockers, calcium blockers, angiotensin-converting enzyme inhibitors, and potassium channel activators; a non-steroidal anti-inflammatory drug (NSAID) selected from the group including, but not limited to acetaminophen, ibuprofen, naproxen, diclofenac, salicylates and indometacin or a COX-2 selective inhibitor selected from the group including, but not limited to rofecoxib, celecoxib, etodolac and meloxicam.

The ratio of Free-B-Ring flavonoids to flavans in the composition of matter can be adjusted based on the indications and the specific requirements with respect to prevention and treatment of a specific disease or condition. Generally, the ratio of Free-B-Ring flavonoids to flavans can be in the range of about 99:1 Free-B-Ring flavonoids: flavans to about 1:99 of Free-B-Ring flavonoids:flavans. In specific embodiments of the present invention, the ratio of Free-B-Ring flavonoids to flavans is selected from the group consisting of approximately 90:10, 80:20, 70:30,60:40, 50:50, 40:60, 30:70, 20:80 and 10:90. In a preferred embodiment of the invention, the ratio of Free-B-Ring flavonoids:flavans in the composition of matter is approximately 85:15. The Free-B-Ring flavonoids and flavans can be synthesized and/or isolated from a single plant or multiple plants. In a preferred embodiment, the Free-B-Ring flavonoids are isolated from a plant or plants in the *Scutellaria* genus of plants and flavans are isolated from a plant or plants in the *Acacia* and *Uncaria* genus of plants.

The present invention further includes methods for treating and preventing diseases and conditions related to platelet aggregation and platelet-induced thrombosis. The method is comprised of administering to a host in need thereof a composition comprising a mixture of Free-B-Ring flavonoids and flavans synthesized and/or isolated from a single plant or multiple plants. The efficacy of this method is demonstrated using purified enzymes, in different cell lines and multiple animal models.

Diseases and conditions related to platelet aggregation and platelet-induced thrombosis that can be prevented and treated according to the method of this invention include, but are not limited to deep vein thrombosis, pulmonary embolism, atherosclerosis, myocardial infarction, thrombosis in cerebral vessels and/or embolism of cerebral vessels leading to cerebrovascular events, thrombosis or peripheral circulation and/or microcirculation resulting in ischemia and infarction, atrial fibrillation that is associated with the stasis of blood and formation of thrombosis in the left atria, thrombogenic sites including artificial implantations such as mechanical heart valves, defibricators, surgical implantations for drug delivery, and artificial hips, joints and other exogenous organs.

The present invention further includes methods for using UP736 as an adjuvant and/or a synergistic, and/or a potentiating agent, said methods comprising administering to a host in need thereof an effective amount of a composition ofmatter comprised of a mixture of at least one Free-B-Ring flavonoid, at least one flavan and at least one agent selected from the group consisting of an injectable anticoagulant, an oral anticoagulant, an antiplatelet agent, an anti-angina agent, a non-steroidal anti-inflammatory drug (NSAID) or a COX-2 selective inhibitor. Examples of injectable anticoagulants include, but are not limited to heparin, dalteparin, enoxaparin and tinzaparin. Examples of oral anticoagulants include, but are not limited to warfarin, vitamin K antagonists and vitamin K reductase inhibitors. Examples of antiplatelet agents include, but are not limited to aspirin, clodipogrel and dipyridamole. Examples of anti-angina drugs include, but not limited to nitrates, beta-blockers, calcium blockers, angiotensin-converting enzyme inhibitors, and potassium channel activators. Non-steroidal anti-inflammatory drugs (NSAIDs) include, but not limited to acetaminophen, ibuprofen, naproxen, diclofenac, salicylates and indometacin. Finally, examples of COX-2 selective inhibitors include, but not limited to rofecoxib, celecoxib, etodolac and meloxicam.

The present invention also includes a method for reducing the standard dose of anti-platelet, anti-coagulant, prophylaxis agents, NSAIDs and COX-2 selective inhibitors to achieve either the equivalent or improved clinical output. The method comprises administering to a host in need thereof an effective amount of a composition comprising a mixture of at least one Free-B-Ring flavonoid and at least one flavan in combination with said anti-platelet, anti-coagulant, prophylaxis agent, NSAID or COX-2 selective inhibitor.

The present invention further includes a composition and a method for using UP736 as an adjuvant and/or a synergistic, and/or a potentiating agent in conjunction with at least one non-steroidal anti-inflammatory drug (NSAID), selected from the group including but not limited to acetaminophen, ibuprofen, naproxen, diclofenac, salicylates, indometacin; and at least one COX-2 selective inhibitor, selected from the group including but not limited to rofecoxib, celecoxib, etodolac, meloxicam. Said composition and method reduces the dose of NSAIDs required to achieve either equivalent or improved clinical output; resulting in a decrease in the side effects associated with the acute or chronic administration these agents and a counteraction or antagonization of the risks of acute or chronic administration of NSAIDs. Said method also provides a means for achieving additional and/or multiple clinical benefits as detailed below. The method comprises administering to a host in need thereof an effective amount of a composition comprising a mixture of Free-B-Ring flavonoids and flavans in combination with at least one NSAID and at least one COX-2 selective inhibitor and a pharmaceutically acceptable carrier.

The present invention also includes a composition and method for decreasing or eliminating the side effects associated with acute or chronic administration of anti-platelet, anti-coagulant, prophylaxis agents, NSAIDs and COX-2 selective inhibitors by the administration of said agent in conjunction with UP736. The method comprises administering to a host in need thereof an effective amount of a composition comprising a mixture of Free-B-Ring flavonoids and flavans in combination with said anti-platelet, anti-coagulant, prophylaxis agent, NSAID or COX-2 selective inhibitor and a pharmaceutically acceptable carrier.

The present invention further includes a method for counteracting or antagonizing the risks associated with acute or chronic administration of anti-platelet, anti-coagulant, prophylaxis agents, NSAIDs and COX-2 selective inhibitors by co-administration of said agent with UP736. The method comprises administering to a host in need thereof an effective amount ofa composition comprising a mixture of Free-B-Ring flavonoids and flavans in combination with said anti-platelet, anti-coagulant, prophylaxis agent or NSAID and a pharmaceutically acceptable carrier.

Finally, the present invention includes methods for achieving additional and/or multiple clinical benefits by the co-administration of anti-platelet, anti-coagulant, prophylaxis agents, NSAIDs and COX-2 selective inhibitors in combination with UP736. As discussed below, UP736 is a potent antioxidant, which regulates the production of the messenger RNA of NFκB and PPAR-γ, leading to the specific down-regulation of TNFα, IL-1β, IL-6 and other pro-inflammatory cytokines, both at the gene expression and protein production levels. The method is comprised of administering to a host in need thereof an effective amount of a composition comprising a mixture of Free-B-Ring flavonoids and flavans synthesized and/or isolated from a single plant or multiple plants in combination with said anti-platelet, anti-coagulant, prophylaxis agent, NSAID or COX-2 selective inhibitor and a pharmaceutically acceptable carrier.

The Free-B-Ring flavonoids, also referred to herein as Free-B-Ring flavones and flavonols, that can be used in accordance with the following invention include compounds illustrated by the following general structure: 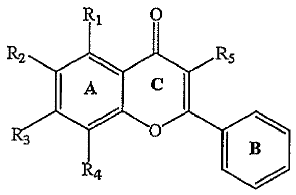 wherein
R₁, R₂, R₃, R₄, and R₅ are independently selected from the group consisting of -H, - OH, -SH, OR, -SR, -NH₂, -NHR, -NR₂, -NR₃⁺X⁻, a carbon, oxygen, nitrogen or sulfur, glycoside of a single or a combination of multiple sugars including, but not limited to aldopentoses, methyl-aldopentose, aldohexoses, ketohexose and their chemical derivatives thereof;
wherein
R is an alkyl group having between 1-10 carbon atoms; and
X is selected from the group ofpharmaceutically acceptable counter anions including, but not limited to hydroxyl, chloride, iodide, sulfate, phosphate, acetate, fluoride, carbonate, etc.

The flavans that can be used in accordance with the following invention include compounds illustrated by the following general structure: 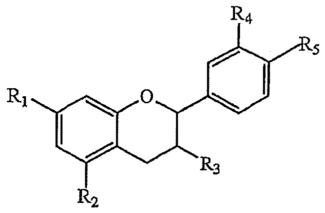 wherein
R₁, R₂, R₃, R₄ and R₅ are independently selected from the group consisting ofH, -OH, -SH, -OCH₃, -SCH₃, -OR, -SR, -NH₂, -NRH, -NR₂, -NR₃⁺X⁻, esters of the mentioned substitution groups, including, but not limited to, gallate, acetate, cinnamoyl and hydroxyl-cinnamoyl esters, trihydroxybenzoyl esters and caffeoyl esters and their chemical derivatives thereof; carbon, oxygen, nitrogen or sulfur glycoside of a single or a combination of multiple sugars including, but not limited to, aldopentoses, methyl aldopentose, aldohexoses, ketohexose and their chemical derivatives thereof; dimer, trimer and other polymerized flavans;
wherein
R is an alkyl group having between 1-10 carbon atoms; and
X is selected from the group of pharmaceutically acceptable counter anions including, but not limited to hydroxyl, chloride, iodide, sulfate, phosphate, acetate, fluoride, carbonate, etc.

The Free-B-Ring flavonoids of this invention may be obtained by synthetic methods and/or extracted from a plant or plants the families of plants including, but not limited to *Annonaceae, Asteraceae, Bignoniaceae, Combretaceae, Compositae, Euphorbiaceae, Labiatae, Lauranceae, Leguminosae, Moraceae, Pinaceae, Pteridaceae, Sinopteridaceae, Ulmaceae* and *Zingiberaceae.* The Free-B-Ring flavonoids can be extracted, concentrated, and purified from the genera of high plants, including but not limited to *Desmos, Achyrocline, Oroxylum, Buchenavia, Anaphalis, Cotula, Gnaphalium, Helichrysum, Centaurea, Eupatorium, Baccharis, Sapium, Scutellaria, Molsa, Colebrookea, Stachys, Origanum, Ziziphora, Lindera, Actinodaphne, Acacia, Derris, Glycyrrhiza, Millettia, Pongamia, Tephrosia, Artocarpus, Ficus, Pityrogramma, Notholaena, Pinus, Ulmus* and *Alpinia.*

The biologically active flavans of this invention may be obtained by synthetic methods and/or extracted from a plant or plants selected from the genus of *Acacia* and/or *Uncaria.* In a preferred embodiment, the *Acacia* plant is selected from the group including, but not limited to *A. catechu, A. concinna, A. farnesiana, A. Senegal, A. speciosa, A. arabica, A. caesia, A. pennata, A. sinuata. A. mearnsii, A. picnantha, A. dealbata, A. auriculiformis, A. holoserecia* and *A. mangium.* In a preferred embodiment, the *Uncaria* plant is selected from the group consisting of *Uncaria gambir, U. lanosa, U. hirsute, U. africana, U. elliptica, U. orientalis, U. attenuate, U. acida, U. homomalla, U. sessilifructus, U. sterrophylla, U. bernaysii, U. sinensis, U. callophylla, U. rhychophylla, U. tomentosa, U. longiflora, U. hirsute, U. cordata,* and *U. borneensis.*

In a preferred embodiment, the Free-B-Ring flavonoids are isolated from a plant or plants in the *Scutellaria* genus of plants and flavans are isolated from a plant or plants in the *Acacia* and *Uncaria* genus ofplants.

As noted above, ratio of Free-B-Ring flavonoids to flavans can be in the range of about 99:1 Free-B-Ring flavonoids:flavans to about 1:99 of Free-B-Ring flavonoids:flavans. In specific embodiments of the present invention, the ratio of Free-B-Ring flavonoids to flavans is selected from the group consisting of approximately 90:10, 80:20, 70:30, 60:40, 50:50,40:60,30:70,20:80 and 10:90. In a preferred embodiment of the invention, the ratio of Free-B-Ring flavonoids:flavans in the composition of matter is approximately 85:15.

It is to be understood that both the foregoing general description and the following detailed description are exemplary and explanatory only and are not restrictive of the invention as claimed.

### BRIEF DESCRIPTION OF THE DRAWINGS

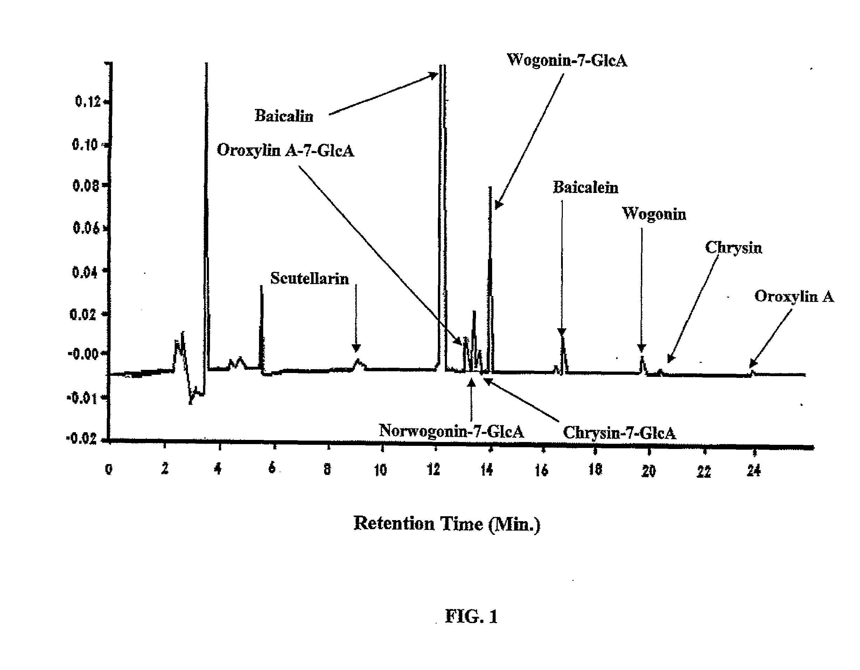

Figure 1 depicts a HPLC chromatogram of a standardized extract isolated from the roots of *S. baicalensis* (lot # RM052302-01) having a Free-B-Ring flavonoid content of 82.2%. As can be seen in the Figure, the following ten compounds were elucidated using HPLC/PDA/MS: baicalin, wogonin-7-glucuronide, oroxylin A 7-glucuronide, baicalein, wogonin, chrysin-7-glucuronide, norwogonin-7-glucuronide, scutellarin, chrysin and oroxylin A.

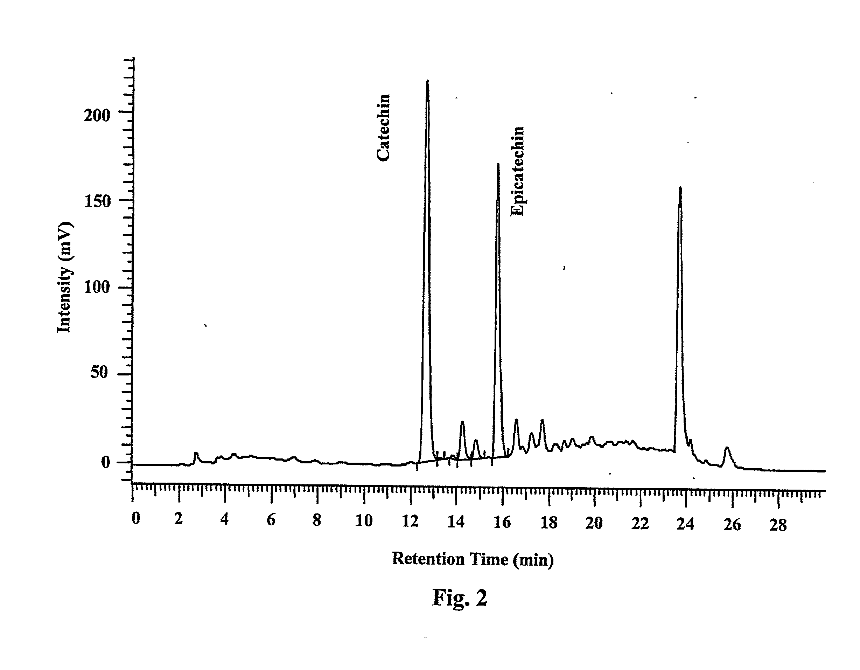

Figure 2 depicts the HPLC chromatogram of the flavans extracted from *A*. *catechu* with 80% MeOH in water.

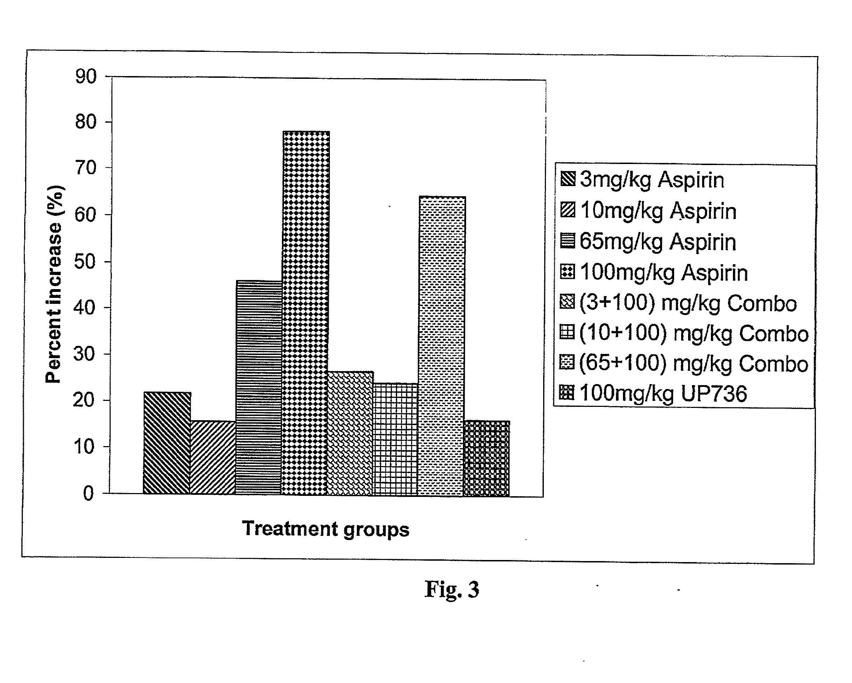

Figure 3 depicts graphically bleeding time and percent increase of bleeding time in treatment groups relative to vehicle control using the combined data from Example 11 (n= 9-10). Average bleeding time and percent increase of bleeding time in treatment groups relative to vehicle control are presented and analyzed using the Student's t-test. Prolongation of bleeding time of treatment groups is expressed as percent increase of bleeding time relative to vehicle control.

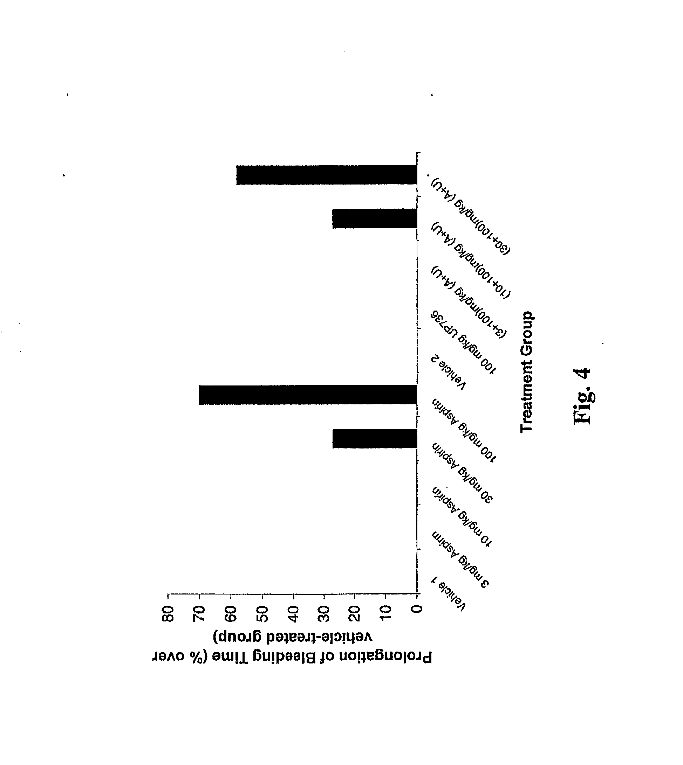

Figure 4 depicts graphically the results from Example 12. In this Example, UP736 was orally administered at a dose of 100 mg/kg, either alone or in combination with aspirin at 3, 10 and 30 mg/kg to groups of 5 ICR derived male mice, weighing 22 ± 2 g, 1 hour before transection of the tip (0.3 mm) of each tail. In addition, aspirin alone at 3, 10, 30 and 100 mg/kg was similarly administered to mice. Prolongation of bleeding time by 50 percent or more (50%) relative to a control group of animals was considered significant.

### DETAILED DESCRIPTION OF THE PREFERRED EMBODIMENT

Various terms are used herein to refer to aspects of the present invention. To aid in the clarification of the description of the components of this invention, the following definitions are provided.

It is to be noted that the term "a" or "an" entity refers to one or more of that entity; for example, a flavonoid refers to one or more flavonoids. As such, the terms "a" or "an", "one or more" and "at least one" are used interchangeably herein.

**"Free-B-Ring Flavonoids"** as used herein are a specific class of flavonoids, which have no substitute groups on the aromatic B-ring, as illustrated by the following general structure: 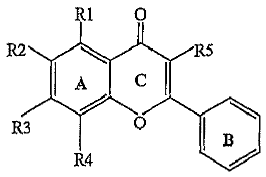 wherein
R₁, R₂, R₃, R₄, and R₅ are independently selected from the group consisting of -H, - OH, -SH, OR, -SR, -NH₂, -NHR, -NR₂, -NR₃⁺X⁻, a carbon, oxygen, nitrogen or sulfur, glycoside of a single or a combination of multiple sugars including, but not limited to aldopentoses, methyl-aldopentose, aldohexoses, ketohexose and their chemical derivatives thereof;
wherein
R is an alkyl group having between 1-10 carbon atoms; and
X is selected from the group of pharmaceutically acceptable counter anions including, but not limited to hydroxyl, chloride, iodide, sulfate, phosphate, acetate, fluoride, carbonate, etc.

**"Flavans"** as used herein refer to a specific class of flavonoids, which can be generally represented by the following general structure: 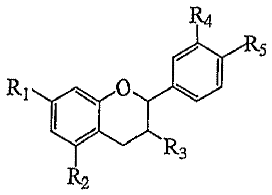 wherein
R₁, R₂, R₃, R₄ and R₅ are independently selected from the group consisting of H, -OH, -SH, -OCH₃, -SCH₃, -OR, -SR, -NH₂, -NRH, -NR₂, -NR₃⁺X⁻, esters of substitution groups, including, but not limited to, gallate, acetate, cinnamoyl and hydroxyl-cinnamoyl esters, trihydroxybenzoyl esters and caffeoyl esters and their chemical derivatives thereof carbon, oxygen, nitrogen or sulfur glycoside of a single or a combination of multiple sugars including, but not limited to, aldopentoses, methyl aldopentose, aldohexoses, ketohexose and their chemical derivatives thereof; dimer, trimer and other polymerized flavans;
wherein
R is an alkyl group having between 1-10 carbon atoms; and
X is selected from the group ofpharmaceutically acceptable counter anions including, but not limited to hydroxyl, chloride, iodide, sulfate, phosphate, acetate, fluoride, carbonate, etc.

**"Therapeutic"** as used herein, includes treatment and/or prophylaxis. When used, therapeutic refers to humans as well as other animals.

**"Pharmaceutically or therapeutically effective dose or amount"** refers to a dosage level sufficient to induce a desired biological result. That result may be the alleviation of the signs, symptoms or causes of a disease or any other alteration of a biological system that is desired.

**"Placebo"** refers to the substitution of the pharmaceutically or therapeutically effective dose or amount dose sufficient to induce a desired biological that may alleviate the signs, symptoms or causes of a disease with a non-active substance.

A **"host"** or **"patient"** is a living subject, human or animal, into which the compositions described herein are administered. Thus, the invention described herein may be used for veterinary as well as human applications and the terms "patient" or "host" should not be construed in a limiting manner. In the case of veterinary applications, the dosage ranges can be determined as described below, taking into account the body weight of the animal.

**"Gene expression"** refers to the transcription of a gene to mRNA.

**"Protein expression"** refers to the translation ofmRNA to a protein.

The present invention relates generally to a composition of matter formulated for use in the prevention and treatment of diseases and conditions related to platelet aggregation and platelet-induced thrombosis. This composition of matter is referred to herein as UP736. The composition of matter is comprised of an individual or a mixture of two specific classes of compounds --Fxee-B-Ring flavonoids and flavans.

The ratio of Free-B-Ring flavonoids to flavans in the composition of matter can be adjusted based on the indications and the specific requirements with respect to prevention and treatment of a specific disease or condition. Generally, the ratio of Free-B-ring flavonoids to flavans can be in the range of about 99:1 Free-B-Ring flavonoids:flavans to about 1:99 of Free-B-Ring flavonoids:flavans. In specific embodiments of the present invention, the ratio of Free-B-Ring flavonoids to flavans is selected from the group consisting of approximately 90:10, 80:20, 70:30, 60:40, 50:50, 40:60, 30:70, 20:80 and 10:90. In a preferred embodiment of the invention, the ratio of Free-B-Ring flavonoids:flavans in the composition of matter is approximately 85:15.

In one embodiment of the present invention, the standardized Free-B-Ring flavonoid extract is comprised of the active compounds with a purity of between 1-99% (by weight) of total Free-B-Ring flavonoids as defined in Examples 3, 4 and 8. Baicalin is the major active component in the extract, which accounts for approximately 50-90% (by weight) of the total Free-B-Ring flavonoids. In a preferred embodiment, the standardized extract contains >70% total Free-B-Ring flavonoids of which >75% of the Free-B-Ring flavonoids is baicalin.

In one embodiment, the standardized flavan extract is comprised of the active compounds with a purity of between 1-99% (by weight) total flavans as defined in Examples 5, 6, and 7. Catechin is the major active component in the extract and accounts for 50-90% (by weight) of the total flavans. In a preferred embodiment, the standardized flavan extract contains >50% total flavans in which >70% of flavans is catechin.

In one embodiment UP736 is produced by mixing either plant extracts as detailed above or synthetic equivalents thereof in a ratio from 99:1 to 1:99 (Free-B-Ring flavonoids: flavans). The preferred ratio of Free-B-Ring flavonoids to flavans is 85:15 Free-B-Ring flavonoids:flavans as defined in Example 9. The concentration of Free-B-Ring flavonoids in UP736 can be from about 1% to 99% and the concentration of flavans in UP736 can be from 99% to 1%. In a preferred embodiment of the invention, the concentration of total Free-B-ring flavonoids in UP736 is approximately 75% with a baicalin content of approximately 60% of total weight of the UP736; and the concentration of total flavans in UP736 is approximately 10% with a catechin content of approximately 9%. In this embodiment, the total active components (Free-B-Ring flavonoids plus flavans) in UP736 are >80% of the total weight.

The present invention includes an evaluation of different compositions of Free-B-Ring flavonoids and flavans using enzymatic and *in vivo* models to optimize the formulation and obtain the desired physiological activity. To date, the Applicant of the current invention is unaware of any reports of a formulation combining only Free-B-Ring-Flavonoids and flavans as the primary biologically active components for the treatment of diseases and conditions. The lack of substitution of one of the aromatic rings of the Free-B-Ring flavonoid plays very important role in making these compounds efficacious. Unlike many other non-steroidal anti-inflammatory drugs (NSAIDs) and natural occurring compounds, Free-B-Ring flavonoids, such as baicalin, have a low polarity aromatic ring on one side of the molecule and high polarity glucuronide and two hydroxyl groups on the other side. This structural arrangement allows these compounds to target tissues and cells. The combination of Free-B-Ring flavonoids with flavans to produce the composition of matter referred to herein as UP736, offers a synergistic and potent modulator of both the COX and LOX pathways of the eicosanoid system

It is clearly demonstrated herein that the combination of Free-B-Ring flavonoids and flavans provides a more balanced modulation of the COX-1 and COX-2 enzymes. For example, aspirin, a COX-1 selective inhibitor, which is more than 150 times selective against COX-1, causes gastrointestinal side effects. Conversely, Vioxx, celebrex and Bextra, which are selective COX-2 inhibitors having 50-200 times more potency against the COX-2 enzyme, do not cause as much gastrointestinal damage, however, these COX-2 selective drugs increase cardiovascular risks.

A profile of the inhibition of COX-1 and COX-2 by the purified component baicalin, which was isolated from *S. baicalensis* showed almost twice the selectivity against COX-2 (the IC₅₀ for COX-1 was determined to be 0.44 µg/mL/unit of enzyme and the IC₅₀ for COX-2 was determined to be 0.28 µg/mL/unit). Whereas, a profile of the inhibition of COX-1 and COX-2 by a composition of matter comprised of greater than 90% catechins isolated from *A*. *catechu,* is almost three times more COX-1 selective. For, catechin, the IC₅₀ of COX-1 inhibition was calculated as 0.11 µg/mL/unit of enzyme and the IC₅₀ for COX-2 was calculated as 0.42 µg/mL/unit.

A combination of a mixture Free-B-Ring flavonoids extracted from the roots of *S*. *baicalensis* and flavans isolated from the bark of *A*. *catechu* in a ratio of 80:20, to obtain a composition of matter, referred to hereinafter as UP736, provides a balanced COX-1 vs COX-2 selectivity of 2:1. This formulation, which provides a balance between the greater COX-2 activity of baicalin and the greater COX-1 activity of catechin, offers optimal modulation of the eicosanoid pathway without the gastrointestional side effects associated with COX-1 selective NSAIDs and cardiovascular risks associated with COX-2 selective inhibitors.

It is also significant that the mechanism of action is completely different between the currently available drugs referenced above and the natural formula - UP736. Aspirin, Vioxx, celebrex and Bextra irreversibly bind to the COX enzyme through covalent bonds to form tightly bound enzyme-inhibitor complexes. Such dramatic interaction completely changes the active site of the enzyme and the side pocket and destroys the enzyme. (Walker et al. (2001) Biochem. 357:709-718). The flavonoids in UP736, on the other hand, inhibit the COX enzyme through a weaker and reversible binding due to their antioxidant properties. In this interactive process, the structure and function of the COX enzyme are not irreversibly altered which results in a much better tolerance and safety profile for UP736.

The inhibition of LOX activity by a flavan extract isolated from *A*. *catechu,* was assessed using a lipoxygenase screening assay *in vitro.* By the addition of flavans to the Free-B-Ring flavonoids, UP736 also inhibits the activity of 5-lipooxygenase (LOX). The inhibition of LOX results in a decrease in the accumulation of phagocytic leukotrienes, which are directly associated with the symptoms of chronic inflammation, and also reduces potential gastrointestinal side effects. It is evident that the combination of Free-B-Ring flavonoids with flavans provides the additional benefit of significantly reducing leukotriene production. This reduction in leukotriene production is by far superior to traditional non-steroidal anti-inflammatory drugs such as ibuprofen in the term of improving efficacy and reducing side effects as discussed in the background section.

The advantages of a formulation comprised of a mixture of Free-B-Ring flavonoid and flavan extracts was also demonstrated by two animal studies, which showed that this novel composition of matter exhibited unexpected synergistic effects. The composition of matter used in these two studies was comprised of a mixture of Free-B-Ring flavonoids obtained from *Oroxylum indicum* seed extract (10.0g) (lot # 040723) having a Free-B-Ring flavonoid -chrysin content of 62.3% and flavans derived *Unicaria gambir* whole plant extract (40.0g) (lot # UG0407-050420) with total catechin content of 32.5%. A combination of above two extracts in a blending ratio of 80:20 provided a formulation called UP736 (50.0 g, Lot#BH-283-14-1). The individual Free-B-Ring flavonoid extract from the seeds of *Oroxylum indicum,* flavan extract from the whole plants of *Unicaria gambir,* and a combination of those extracts (UP736) were administrated orally in a dosage of 100 mg/kg using an indomethacin control in an acute inflammation animal model, Mouse Ear Swelling Test. The inhibition of ear swelling (50.8% inhibition) from UP736 was significantly better than the same dose of individual components 36.5% *Uncaria gambir* extract, and 31.7% from *Oroxylum indicum* extract, respectively.

In another *in vivo* arachidonic acid-induced mouse ear swelling inhibition assay, synergistic effects were also observed in a formulation called UP736-K, which was blended in a ratio of 9:1 with a Free-B-Ring flavonoid extract from the roots *of S. baicalensis* containing 25% baicalin and a 40% catechin extract from the whole plant of *Uncaria gambir.* UP736-K contained 24% baicalin and 4% catechins. The individual Free-B-Ring flavonoid extract from the roots of *S*. *baicalensis,* flavan extract from whole plants of *Unicaria gambir,* and a combination of those extracts (UP736-K) were administrated orally in a dosage of 100 mg/kg using an indomethacin control. UP736- K showed statistically significant improvement in reducing edema relative to each of the individual extracts.

Additionally, due to the different biological availability, i.e. rate and percentage of biologically active compounds penetrating the epithelial cell membrane and the local concentrations of biologically active compounds, the combination of the two different types of compounds (higher polarity flavans vs. lower polarity Fxee-B-Ring flavonoids) offers both quick, on-site COX/LOX inhibition by the biologically active flavans, together with longer lasting modulation of COX/LOX pathway by the biologically active Free-B-Ring flavonoids. It takes about two hours after oral administration for Free-B-Ring flavonoids in UP736 to reach efficacious concentrations. However, serum concentration of the Free-B-Ring flavonoids will remain above therapeutic levels for approximately 10 hours after oral administration. To compensate for the lack of quick bioavailability from Free-B-Ring flavonoids, the formulation of catechin type flavans offers a complimentary benefit. Studies of the bioavailability of catechins, quercetin, and epigallocatechin-3-gallate (Kao et al. (2000) Endocrinology 141(3):980-987; Koga and Meydani (2001) Am. J. Clin. Nutr. 73:941-948; Lee et al. (2002) Cancer Epidemiol. Biomarker Prevention 11:1025-1032) show that the Cₘₐₓ and Tₘₐₓ of catechin occur quickly (about 45 minutes) and the half-life was reported to be 2 hours. Therefore, by combining Free-B-Ring-flavonoids with flavans, the quickly penetrating catechins reach efficacious serum concentrations in about 0.5 hour after oral administration. When the catechin concentration drops, the second active component, the Free-B-Ring flavonoids reach bioactive concentrations that will last up to 12 hours after oral administration. In conclusion, the UP736 formulation is designed to have quick on-site COX/LOX effects resulting from the flavans, such as catechin and longer lasting effects resulting from the Free-B-Ring flavonoids, such as baicalin. Such synergistic and complimentary effects will also be realized via topical delivery of the formula.

Finally, in a preferred embodiment of the formulation, which has significant amounts of Free-B-Ring flavonoids (80% by weight) with comparatively lower concentration of flavans (20% by weight), the more potent anti-oxidative flavans will function both as natural preservatives against oxidative degradation of the Free-B-Ring flavonoids and to neutralize and buffer the composition allowing delivery of the major active components -- the Free-B-Ring flavonoids at the optimum pH and ionization conditions. Catechin contains four phenolic hydroxyl groups, which makes this compound more acidic and sensitive to oxidative stress. The extremely high Oxygen Radical Absorption Capacity (ORAC at 20,000) of catechin demonstrates its antioxidant properties. Based upon the stress test of pure catechin under varying conditions, such as pH, existence of H₂O₂ and metal ions, it was determined (data is available but not shown) that catechin is stable under neutral conditions at both 4°C and 40°C, but not under basic conditions or when exposed to metal ions, such as Fe³⁺. Even under weakly basic conditions (pH=7.5) catechin decomposes. However, it can be preserved by a number of preservatives, including but not limited to stannous chloride (SnCl₂), sodium bisulfate/metabisulfite (SBS), and other preservatives.

Included in the present invention is a novel composition of matter comprised of a mixture of at least one Free-B-Ring flavonoid, at least one flavan and at least one agent selected from the group consisting of an injectable anticoagulant, selected from the group including, but not limited to heparin, dalteparin, enoxaparin and tinzaparin; an oral anticoagulant, selected from the group including, but not limited to warfarin, vitamin K antagonists and vitamin K reductase inhibitors; an antiplatelet agent, selected from the group including, but not limited to aspirin, clodipogrel and dipyridamole; an anti-angina drug, selected from the group including, but not limited to nitrates, beta-blockers, calcium blockers, angiotensin-converting enzyme inhibitors, and potassium channel activators; a non-steroidal anti-inflammatory drug (NSAID) selected from the group including, but not limited to acetaminophen, ibuprofen, naproxen, diclofenac, salicylates and indometacin or a COX-2 selective inhibitor selected from the group including, but not limited to rofecoxib, celecoxib, etodolac and meloxicam

The present invention further includes methods for treating and preventing diseases and conditions related to platelet aggregation and platelet-induced thrombosis. Thrombosis is the unwanted formation of blood clots that may be venous or arterial. UP736 can be utilized as an anti-platelet, anti-coagulant and prophylaxis agent for the prevention and treatment of the above mentioned diseases and conditions. The method is comprised of administering to a host in need thereof an effective amount of a composition comprising a mixture of Free-B-Ring flavonoids and flavans synthesized and/or isolated from a single plant or multiple plants.

Diseases and conditions related to platelet aggregation and platelet-induced thrombosis that can be prevented and treated according to the method of this invention include, but are not limited to deep vein thrombosis, pulmonary embolism, atherosclerosis, myocardial infarction, thrombosis in cerebral vessels and/or embolism of cerebral vessels leading to cerebrovascular events, thrombosis or peripheral circulation and/or microcirculation resulting in ischemia and infarction, atrial fibrillation that is associated with the stasis of blood and formation of thrombosis in the left atria, thrombogenic sites including artificial implantations such as mechanical heart valves, defibricators, surgical implantations for drug delivery, and artificial hips, joints and other exogenous organs.

The present invention further includes methods for using UP736 as an adjuvant and/or a synergistic, and/or a potentiating agent, said methods comprising administering to a host in need thereof an effective amount of a composition of matter comprised of a mixture of at least one Free-B-Ring flavonoid, at least one flavan and at least one agent selected from the group consisting of an injectable anticoagulant, an oral anticoagulant, an antiplatelet agent, an anti-angina agent, a non-steroidal anti-inflammatory drug (NSAID) or a COX-2 selective inhibitor. Examples of injectable anticoagulants include, but are not limited to heparin, dalteparin, enoxaparin and tinzaparin. Examples of oral anticoagulants include, but are not limited to warfarin, vitamin K antagonists and vitamin K reductase inhibitors. Examples of antiplatelet agents include, but are not limited to aspirin, clodipogrel and dipyridamole. Examples of anti-angina drugs include, but not limited to nitrates, beta-blockers, calcium blockers, angiotensin-converting enzyme inhibitors, and potassium channel activators. Non-steroidal anti-inflammatory drugs (NSAIDs) include, but not limited to acetaminophen, ibuprofen, naproxen, diclofenac, salicylates and indometacin. Finally, examples of COX-2 selective inhibitors include, but not limited to rofecoxib, celecoxib, etodolac and meloxicam.

The present invention also includes a method for reducing the standard dose of anti-platelet, anti-coagulant, prophylaxis agents, NSAIDs and COX-2 selective inhibitors to achieve either the equivalent or improved clinical output. The method comprises administering to a host in need thereof an effective amount of a composition comprising a mixture of at least one Free-B-ring flavonoid and at least one flavan, either synthesized and/or isolated from a single plant or multiple plants and a pharmaceutically acceptable carrier in combination with said anti-platelet, anti-coagulant, prophylaxis agent, NSAID or COX-2 selective inhibitors.

UP736 is a natural product derived from two traditional plants that contain antioxidants and other naturally occurring dietary compounds that aid the body in multiple ways. UP736 is not a selective COX-2 inhibitor, but rather is 2.25 times more selective against COX-1 vs. COX-2, and in addition naturally inhibits 5-lipoxygenase (LOX), which regulates the pathway that produces multiple vasodilating and chemotactic leukotrienes. The naturally occurring inhibitory activity of UP736 toward COX-2 as assayed by enzymatic inhibition is approximately 50-400 times less effective in comparison to the highly selective COX-2 drugs,--Rofecoxib and Celecoxib-- as shown in the Table 1.

**Table 1. COX-2 Activity of UP736 Relative to Known COX-2 Inhibitors**

| **Compound** | **COX-2 Selectivity Relative COX-1** |
|---|---|
| **Rofecoxib** | **250** |
| **Celecoxib** | **30** |
| **Licofelone** | **1** |
| **UP736** | **0.44** |
| **Indomethacin** | **0.016** |
| **Aspirin** | **0.006** |

UP736 is also a potent antioxidant, which naturally regulates the production of the messenger RNA of NFκB and PPAR-γ, leading to the specific down-regulation of TNFα, IL-1β, IL-6 and other pro-inflammatory cytokines at both the gene expression and protein production levels.

The present invention further includes a composition and a method for using UP736 as an adjuvant and/or a synergistic, and/or a potentiating agent in conjunction with at least one non-steroidal anti-inflammatory drug (NSAID), including but not limited to acetaminophen, ibuprofen, naproxen, diclofenac, salicylates, indometacin; and at least one COX-2 selective inhibitors, including but not limited to rofecoxib, celecoxib, etodolac, meloxicam. Said composition and method reduces the dose of NSAIDs required to achieve either equivalent or improved clinical output; resulting in a decrease in the side effects associated with the acute or chronic administration of NSAIDs and a counteraction or antagonization the risks of acute or chronically administration of NSAIDs. Said method and composition also achieves additional and/or multiple clinical benefits resulting from the specific down-regulation of TNFα, IL-1β, IL-6 and other pro-inflammatory cytokines as described above.

The present invention also includes a composition and method for decreasing or eliminating the side effects associated with acute or chronic administration of anti-platelet, anti-coagulant, prophylaxis agents, NSAIDs and COX-2 selective inhibitors by the administration of said agent in combination with UP736. The method comprises administering to a host in need thereof an effective amount of a composition comprising a mixture of Free-B-Ring flavonoids and flavans synthesized and/or isolated from a single plant or multiple plants in combination with said anti-platelet, anti-coagulant, prophylaxis agent, NSAID or COX-2 selective inhibitor and a pharmaceutically acceptable carrier.

The present invention further includes a method for counteracting or antagonizing the risks associated with acute or chronic administration of anti-platelet, anti-coagulant, prophylaxis agents, NSAIDs and COX-2 selective inhibitors by co-administration of said agent with UP736. The method comprises administering to a host in need thereof an effective amount of a composition comprising a mixture of Free-B-Ring flavonoids and flavans synthesized and/or isolated from a single plant or multiple plants in combination with said anti-platelet, anti-coagulant, prophylaxis agent, NSAID or COX-2 selective inhibitor and a pharmaceutically acceptable carrier.

Finally, the present invention includes methods for achieving additional and/or multiple clinical benefits by the co-administration of anti-platelet, anti-coagulant, prophylaxis agents, NSAIDs and COX-2 selective inhibitors in combination with UP736. As noted above, UP736 is a potent antioxidant, which regulates the production of the messenger RNA of NFκB and PPAR-γ, leading to the specific down-regulation of TNFα, IL-1β, IL-6 and other pro-inflammatory cytokines, both at the gene expression and protein production levels. The method is comprised of administering to a host in need thereof an effective amount of a composition comprising a mixture of Free-B-Ring flavonoids and flavans synthesized and/or isolated from a single plant or multiple plants in combination with said anti-platelet, anti-coagulant, prophylaxis agent, NSAID or COX-2 selective inhibitor and a pharmaceutically acceptable carrier.

The present invention is directed toward therapeutic compositions comprising the therapeutic agents of the present invention. The therapeutic agents of the instant invention can be administered by any suitable means, including, for example, parenteral, topical, oral or local administration, such as intradermally, by injection, or by aerosol. The particular mode of administration will depend on the condition to be treated. It is contemplated that administration of the agents of the present invention may be via any bodily fluid, or any target or any tissue accessible through a body fluid. In the preferred embodiment of the invention, the agent is administered by oral dosage. Such delivery can be locally administered to any affected area. A therapeutic composition can be administered in a variety of unit dosage forms depending upon the method of administration. For example, unit dosage forms suitable for oral administration of an animal include powder, tablets, pills and capsules. Preferred delivery methods for a therapeutic composition of the present invention include intravenous administration and local administration by, for example, injection or topical administration. A therapeutic reagent of the present invention can be administered to any animal, preferably to mammals, and more preferably to humans.

For particular modes of delivery, a therapeutic composition of the present invention can be formulated so as to include other components such as a pharmaceutically acceptable excipient, an adjuvant, and/or a carrier. For example, compositions of the present invention can be formulated in an excipient that the animal to be treated can tolerate. Examples of such excipients, include but are not limited to cellulose, silicon dioxide, dextrates, sucrose, sodium starch glycolate, calcium phosphate, calcium sulfate, water, saline, Ringer's solution, dextrose solution, mannitol, Hank's solution, and other aqueous physiologically balanced salt solutions. Nonaqueous vehicles, such as fixed oils, sesame oil, ethyl oleate, or triglycerides may also be used. Other useful formulations include suspensions containing viscosity-enhancing agents, such as sodium carboxymethylcellulose, sorbitol, or dextran. Excipients can also contain minor amounts of additives, such as substances that enhance isotonicity and chemical stability. Examples of buffers include phosphate buffer, bicarbonate buffer, Tris buffer, histidine, citrate, and glycine, or mixtures thereof, while examples of preservatives include thimerosal, m- or o-cresol, formalin and benzyl alcohol. Standard formulations can either be liquid injectables or solids, which can be taken up in a suitable liquid as a suspension or solution for injection. Thus, in a non-liquid formulation, the excipient can comprise dextrose, human serum albumin, preservatives, etc., to which sterile water or saline can be added prior to administration.

In one embodiment of the present invention, the composition can also include an adjuvant or a carrier. Adjuvants are typically substances that generally enhance the function of the formula in preventing and treating indications related to COX & LOX pathways. Suitable adjuvants include, but are not limited to, Freund's adjuvant; other bacterial cell wall components; aluminum-based salts; calcium-based salts; silica; boron, histidine, glucosamine sulfates, Chondroitin sulfate, copper gluconate, polynucleotides; vitamin D, vitamin K, toxoids; shark and bovine cartilage; serum proteins; viral coat proteins; other bacterial-derived preparations; gamma interferon; block copolymer adjuvants, such as Hunter's Titermax adjuvant (Vaxcel.TM., Inc. Norcross, Ga.); Ribi adjuvants (available from Ribi ImmunoChem Research, Inc., Hamilton, Mont.); and saponins and their derivatives, such as Quil A (available from Superfos Biosector A/S, Denmark). Carriers are typically compounds that increase the half-life of a therapeutic composition in the treated animal. Suitable carriers include, but are not limited to, polymeric controlled release formulations, biodegradable implants, liposomes, bacteria, viruses, oils, esters, and glycols.

One embodiment of the present invention is a controlled release formulation that is capable of slowly releasing a composition of the present invention into an animal. As used herein, a controlled release formulation comprises a composition of the present invention in a controlled release vehicle. Suitable controlled release vehicles include, but are not limited to, biocompatible polymers, other polymeric matrices, capsules, microcapsules, microparticles, bolus preparations, osmotic pumps, diffusion devices, liposomes, lipospheres, and transdermal delivery systems. Other controlled release formulations of the present invention include liquids that, upon administration to an animal, form a solid or a gel in situ. Preferred controlled release formulations are biodegradable (i.e., bioerodible).

Once the therapeutic composition has been formulated, it may be stored in sterile vials as a solution, suspension, gel, emulsion, solid, or dehydrated or lyophilized powder; or directly capsulated and/or tableted with other inert carriers for oral administration. Such formulations may be stored either in a ready to use form or requiring reconstitution immediately prior to administration. The manner of administering formulations containing the compositions for systemic delivery may be via oral, subcutaneous, intramuscular, intravenous, intranasal or vaginal or rectal suppository.

The amount of the composition that will be effective in the treatment of a particular disorder or condition will depend on the nature of the disorder of condition, which can be determined by standard clinical techniques. In addition, *in vitro* or *in vivo* assays may optionally be employed to help identify optimal dosage ranges. The precise dose to be employed in the formulation will also depend on the route of administration, and the seriousness or advancement of the disease or condition, and should be decided according to the practitioner and each patient's circumstances. Effective doses may be extrapolated from dose-response curved derived from in vitro or animal model test systems. For example, an effective amount of the composition can readily be determined by administering graded doses of the composition and observing the desired effect.

The method of treatment according to this invention comprises administering internally or topically to a patient in need thereof a therapeutically effective amount of the composition comprised of a mixture of Free-B-Ring flavonoids and flavans or a mixture of at least one Free-B-Ring flavonoid, one flavans and one agent selected from the group consisting of an injectable anticoagulant, an oral anticoagulant, an antiplatelet agent, an anti-angina drug, a non-steroidal anti-inflammatory drug (NSAID) or a COX-2 selective inhibitor. The purity of the mixture includes, but is not limited to 0.01% to 100%, depending on the methodology used to obtain the compound(s). In a preferred embodiment, doses of the mixture of Free-B-ring flavonoids and flavans and pharmaceutical compositions containing the same are an efficacious, nontoxic quantity generally selected from the range of 0.01 to 200 mg/kg of body weight. Persons skilled in the art using routine clinical testing are able to determine optimum doses for the particular ailment being treated

A general method for preparing the extracts is described in Example 1. The extraction process yields an organic and an aqueous extract for each species examined. The results of the extraction of various species are set forth in Table 2. In order to efficiently identify active compounds from plant extracts, a high throughput fractionation process was used, as described in Example 2. Briefly, the active organic and aqueous extracts were fractionated using two different methodologies, respectively. The fractions were collected in a 96 deep well plate. Each of the fractions was then tested for its biological activity.

The separation, purification and identification of the active Free-B-Ring flavonoids present in the organic extract of *Scutellaria orthocalyx* is described in Example 3. With reference to Figure 1, ten compounds were elucidated, with Baicalin being identified as the major active component.

Example 4 and Table 3 set forth the content and quantity of the Free-B-Ring flavonoids in five active plant extracts from three different species of plants. The Free-B-Ring flavonoids are present in much greater amounts in the organic extracts verses the aqueous extracts.

The separation, purification and identification of the active components present in the organic extract of *Acacia catechu* is described in Example 5. Using the methodology described in Example 5, catechin and epicatechin were identified as the two major active compounds in the organic extract from the roots of *Acacia catechu,* having IC₅₀values of 5-7 µg/mL. HPLC quantification of the active extracts from *Acacia catechu* and *Unicaria gambir* is described in Example 6. The results are set forth in Table 4 which shows that the flavan content in the organic and aqueous extracts of *A catechu,* as determined by HPLC, is 30.4% and 1.0%, respectively. Example 7 describes a general method for the preparation of a standardized extract from *Acacia.* In this example, flavans from *A*. *catechu* were extracted with different solvent systems. The results are set forth in Table 5. The improved method of this invention comprises: extraction of the ground biomass of a plant containing flavans with an organic solvent or a combination of organic solvent(s) and/or water; neutralization and concentration of the neutralized extract; and purification of said extract by recrystallization and/or chromatography. It can be seen from Table 5, that 80% methanol in water is one of the preferred solvents for extraction of flavans from *Acacia* plants. As provided above, these flavans can be isolated from the *Acacia* and *Unicaria* genus of plants. The method of this invention can be extended to the isolation of these compounds from any plant source containing these compounds.

Example 8 describes a general method for the preparation of a standardized extract from various *Scutellaria* species. In Example 8, Free-B-Ring flavonoids from two *Scutellaria* species were extracted with different solvent systems. The results are set forth in Tables 6 and 7. The method of this invention comprises: extraction of the ground biomass of a plant containing Free-B-Ring flavonoids with single or combination of organic solvents and/or water; neutralization and concentration of the neutralized extract; and purification of said extract by recrystallization and/or chromatography. As provided above, these Free-B-Ring flavonoids can be isolated from the genera of more than twenty plant families. The method of this invention can be extended to the isolation of these compounds from any plant source containing these compounds.

Example 9 describes a general method for preparation of the UP736 composition, which is comprised of a proprietary blending of two standardized extracts, containing Free-B-ring flavonoids and flavans, respectively. In the general method set forth in Example 9 the composition is prepared using two standardized extracts isolated from *Acacia* and *Scutellaria,* respectively, together with or without excipients. The *Acacia* extract used in Example 9 contained >60% total flavans, as catechin and epicatechin, and the *Scutellaria* extract contained >70% Free-B-ring flavonoids, which was primarily baicalin. The *Scutellaria* extract contained other minor amounts of Free-B-ring flavonoids as set forth in Table 8. One or more excipients are optionally added to the composition of matter. The amount of excipient added can be adjusted based on the actual active content of each ingredient desired. A blending table for each individual batch ofpro duct must be generated based on the product specification and QC results for individual batch of ingredients. Additional amounts of active ingredients in the range of 2-5% are recommended to meet the product specification. Example 9 illustrates a blending table that was generated for one batch of UP736 (Lot#G1702). Different blending ratios of the formulated UP736 product were also prepared tested for their biological activity.

Example 10 demonstrates the synergistic effect that a composition comprised of a mixture of UP736 and aspirin has on the inhibition of arachidonic acid induced platelet aggregation. The results are set forth in Tables 9 and 10, which demonstrate that while UP736 alone had little anti-aggregatory activity at concentrations up to 10 µM, the anti-aggregatory of aspirin was significantly increased at dosages as low as 0.007 µM of UP736.

Lowering the dose of aspirin could, theoretically, lower the risk of bleeding complications. However, very low-dose aspirin may not be efficacious in treating or preventing certain diseases and conditions. For example, for long-term treatment of acute myocardial infarction, the effect of doses less than 75 mg daily are not clear (Hennekens (2002) Am. J. Manag. Care 8(22 Suppl):S691-700). In addition, aspirin resistance occurs in individuals who do not respond to low doses of aspirin (Patrono (2005) Thromb. Haemost. 8:1597-602). The current invention solves the problem by using UP736 to potentiate the anti-platelet aggregation activity dramatically conferred by low doses of aspirin.

Example 11 illustrates that UP736 alone and in combination with aspirin has little effect on bleeding time. The results are set forth in Tables 11-14.

Note that throughout this application various citations are provided. Each citation is specifically incorporated herein in its entirety by reference.

The following examples are provided for illustrative purposes only and are not intended to limit the scope of the invention.

### EXAMPLES

The following examples are provided for illustrative purposes only and are not intended to limit the scope of the invention.

### Example 1. Preparation of Organic and Aqueous Extracts from Acacia, Uncaria and Scutellaria Plants

Plant material from *Acacia catechu* (L) *Willd.* barks, *Uncaria hirsute* aerial parts, *Uncaria sinensis* aerial parts, *Uncaria tomentosa* barks, *Scutellaria orthocalyx* roots, *Scutellaria baicalensis* roots or *Scutellaria lateriflora* whole plant was ground to a particle size of no larger than 2 mm. Dried ground plant material (60 g) was then transferred to an Erlenmeyer flask and methanol:dichloromethane (1:1) (600 mL) was added. The mixture was shaken for one hour, filtered and the biomass was extracted again with methanol: dichloromethane (1:1) (600 mL). The organic extracts were combined and evaporated under vacuum to provide the organic extract (see Table 2 below). After organic extraction, the biomass was air dried and extracted once with ultra pure water (600 mL). The aqueous solution was filtered and freeze-dried to provide the aqueous extract (see Table 2 below).

**Table 2. Yield of Organic and Aqueous Extracts of Acacia, Uncaria and Scutellaria Species**

| **Plant Source** | **Amount** | **Organic Extract** | **Aqueous Extract** |
|---|---|---|---|
| *Acacia catechu* barks | 60 g | 27.2 g | 10.8 g |
| *Scutellaria orthocalyx* roots | 60 g | 4.04 g | 8.95 g |
| *Scutellaria baicalensis* roots | 60 g | 9.18 g | 7.18 g |
| *Scutellaria lateriflora* (whole plant) | 60 g | 6.54 g | 4.08 g |
| *Uncaria hirsute* aerial parts | 60 g | 2.41 g | 0.90 g |
| *Uncaria sinensis* aerial parts | 60 g | 3.94 g | 1.81 g |
| *Uncaria tomentosa* barks | 60 g | 6.47 g | 2.31 g |

### Example 2. HTP Fractionation of Active Extracts

Organic extract (400 mg) from active plant was loaded onto a prepacked flash column. (2 cm ID x 8.2 cm, 10g silica gel). The column was eluted using a Hitachi high throughput purification (HTP) system with a gradient mobile phase of (A) 50:50 BtOAc:hexane and (B) methanol from 100% A to 100% B in 30 minutes at a flow rate of 5 mL/min. The separation was monitored using a broadband wavelength UV detector and the fractions were collected in a 96-deep-well plate at 1.9 mL/well using a Gilson fraction collector. The sample plate was dried under low vacuum and centrifugation. DMSO (1.5 mL) was used to dissolve the samples in each cell and a portion (100 µL) was taken for the BIOLOGICAL inhibition assay.

Aqueous extract (750 mg) from active plant was dissolved in water (5 mL), filtered through a 1 µm syringe filter and transferred to a 4 mL High Pressure Liquid Chromatography HPLC) vial. The solution was then injected by an autosampler onto a prepacked reverse phase column (C-18,15 µm particle size, 2.5 cm ID x 10 cm with precolumn insert). The column was eluted using a Hitachi high throughput purification (HTP) system with a gradient mobile phase of (A) water and (B) methanol from 100% A to 100% B in 20 minutes, followed by 100% methanol for 5 minutes at a flow rate of 10 mL/min. The separation was monitored using a broadband wavelength UV detector and the fractions were collected in a 96-deep-well plate at 1.9 mL/well using a Gilson fraction collector. The sample plate was freeze-dried. Ultra pure water (1.5 mL) was used to dissolve samples in each cell and a portion (100 µL) was taken for the biological inhibition assay.

### Example 3. Isolation and Purification of the Active Free-B-Ring Flvonoids from the Organic Extract of Scutellaria

The organic extract (5 g) from the roots of *Scutellaria orthocalyx,* isolated as described in Example 1, was loaded onto prepacked flash column (120 g silica, 40 µm particle size 32-60 µm, 25 cm x 4 cm) and eluted with a gradient mobile phase of (A) 50:50 EtOAc:hexane and (B) methanol from 100% A to 100% B in 60 minutes at a flow rate of 15 mL/min. The fractions were collected in test tubes at 10 mL/ftaction. The solvent was evaporated under vacuum and the sample in each fraction was dissolved in 1 mL of DMSO and an aliquot of 20 µL was transferred to a 96 well shallow dish plate and tested for biological activity (data not shown). Based on the biological assay results, active fractions #31 to #39 were combined and evaporated. Analysis by HPLC/PDA and LC/MS showed a major compound with a retention times of 8.9 minutes and a MS peak at 272 m/e. The product was further purified on a C18 semi-preparation column (25 cm x 1 cm), with a gradient mobile phase of (A) water and (B) methanol, over a period of 45 minutes at a flow rate of 5 mL/minute. Eighty-eight fractions were collected to yield 5.6 mg of light yellow solid. Purity was determined by HPLC/PDA and LC/MS, and comparison with standards and NMR data. ¹H NMR: δ ppm. (DMSO-d6) 8.088 (2H, m, H-3',5'), 7.577 (3H, m, H-2',4',6'), 6.932 (1H, s, H-8), 6.613 (1H, s, H-3). MS: [M+1]+ = 271m/e. The compound was identified as baicalin.

Using preparative C-18 column chromatography, other Free-B-Ring flavonoids were isolated and identified using a standardized extract isolated from the roots of *Scutellaria baicalensis* (lot # RM052302-01), having a Free-B-Ring flavonoid content of 82.2%. Eleven structures were elucidated using HPLC/PDA/MS as illustrated in Figure 1. With reference to Figure 1, the eleven compounds identified were baicalin, wogonin-7-glucuronide, oroxylin A 7-glucuronide, baicalein, wogonin, chrysin-7-glucuronide, 5-methyl-wogonin-7-glucuronide, scutellarin, norwogonin, chrysin and oroxylin A.

### Example 4. HPLC Quantification of Free-B-Ring Flavonoids in Active Extracts Isolated from Scutellaria orthocalyx (roots), Scutellaria baicalensis (roots) and Oroxylum indicum (seeds)

The presence and quantity of Free-B-Ring flavonoids in five active extracts isolated from three different plant species have been confirmed and are set forth in the Table 3. The Free-B-Ring flavonoids were quantitatively analyzed by HPLC using a Luna C-18 column (250 x 4.5 mm, 5 µm) a using 1% phosphoric acid and acetonitrile gradient from 80% to 20% in 22 minutes. The Free-B-Ring flavonoids were detected using a UV detector at 254 nm and identified based on retention time by comparison with Free-B-Ring flavonoid standards.

**Table 3. Free-B-Ring Flavonoid Content in Active Plant Extracts**

| **Active Extracts** | **Weight of Extract** | **% Extractible from BioMass** | **Total amount of Free-B-Ring Flavonoids** | **% Free-B-Ring Flavonoids in Extract** |
|---|---|---|---|---|
| *Scutellaria. orthocalyx* | 8.95 g | 14.9% | 0.2 mg | 0.6% |
| (aqueous extract) | | | | |
| *S. orthocalyx* | 3.43 g | 5.7% | 1.95 mg | 6.4% |
| (organic extract) | | | | |
| *S. baicalensis* | 7.18 g | 12.0% | 0.03 mg | 0.07% |
| (aqueous extract) | | | | |
| *S. baicalensis* | 9.18 g | 15.3% | 20.3 mg | 35.5% |
| (organic extract) | | | | |
| *Oroxylum indicum* | 6.58 g | 11.0% | 0.4 mg | 2.2% |
| (organic extract) | | | | |

### Example 5. Isolation and Purification of Active Compounds from the Organic Extract of Acacia catechu

The organic extract (5 g) from the roots of *A*. *catechu,* isolated as described in Example 1, was loaded onto prepacked flash column (120 g silica, 40 µm particle size 32-60 µm, 25 cm x 4 cm) and eluted with a gradient mobile phase of (A) 50:50 EtOAc:hexane and (B) methanol from 100% A to 100% B in 60 minutes at a flow rate of 15 mL/min. The fractions were collected in test tubes at 10 mL/fraction. The solvent was evaporated under vacuum and the sample in each fraction was dissolved in DMSO (1 mL) and an aliquot of 20 µL was transferred to a 96 well shallow dish plate and tested for biological activity (data not shown). Based upon the biological assay results, active fractions #32 to #41 were combined and evaporated to yield 2.6 g of solid. Analysis by HPLC/PDA and LC/MS showed two major compounds with retention times of 15.8 and 16.1 minutes, respectively. The product was further purified on a C18 semi-preparatory column (25 cm x 1 cm), loaded with 212.4 mg of product and eluted with a gradient mobile phase of (A) water and (B) acetonitrile (ACN), over a period of 60 minutes at a flow rate of 5 mL/minute. Eighty-eight fractions were collected and two active compounds were isolated. Compound 1 (11.5 mg) and Compound 2 (16.6 mg). Purity was determined by HPLC/PDA and LC/MS data by comparison with standards (catechin and epicatechin) and NMR data.

**Compound 1**. ¹³C NMR: δ ppm (DMSO-d6) 27.84 (C4), 66.27 (C3), 80.96 (C2), 93.78 (C9), 95.05 (C7), 99.00 (C5), 114.48 (C12), 115.01 (C15), 118.36 (C16), 130.55 (C11), 144.79 (C14), 155.31 (C6), 156.12 (C10), 156.41 (C8). ¹H NMR: δ ppm. (DMSO-d6) 9.150 (1H, s, OH), 8.911 (1H,s, OH), 8.835 (1H, s, OH), 8.788 (1H, s, OH), 6.706 (1H, d, J=2 Hz, H2'), 6.670 (1H, d, J=8.0 Hz, H-6'), 6.578 (1H, dd, J=2, 8 Hz, H-5'), 5.873 (1H, d, J=2 Hz, H8), 5.670 (1H, d, J=2 Hz, H6), 4.839 (1H, d, J=4 Hz, OH), 4.461 (1H, d, J=7.3 Hz, H2), 3.798 (1H, m, H3), 2.625 (1H, m, H4b), 2.490 (1H, m, H4a). MS: [M+1]⁺ = 291 m/e. This compound was identified as catechin.

**Compound 2.** ¹³C NMR: δ ppm (DMSO-d6) 28.17 (C4), 64.87 (C3), 78.02 (C2), 94.03 (C9), 95.02 (C7), 98.44 (C5), 114.70 (C12), 114.85 (C15), 117.90 (C16), 130.56 (C11), 144.39 (C14), 155.72 (C6), 156.19 (C10), 156.48 (C8). ¹H NMR: δ ppm. (DMSO-d6) 9.083 (1H, s, OH), 8.873 (1H,s, OH), 8.777 (1H, s, OH), 8.694 (1H, s, OH), 6.876 (1H, d, J=2 Hz, H2'), 6.646 (2H, s, H-5', 6'), 5.876 (1H, d, J=2 Hz, H8), 5.700 (1H, d, J=2 Hz, H6), 4.718 (1H, s, OH), 4.640 (1H, d, J=4.5 Hz, H2), 3.987 (1H, d, J=4.5 Hz, H3), 2.663 (1H, dd, J=4.6, 6.3 Hz, H4b), 2.463 (1H,dd, J=4.6, 6.3 Hz, H4a). MS: [M+1]⁺ = 291 m/e. This compound was identified as epicatechin.

### Example 6. HPLC Quantification of Active Extracts from Acacia catechu and Unicaria gambir

The flavan content in the organic and aqueous extracts isolated from heart woods of *Acacia catechu* quantified by HPLC using a PhotoDiode Array detector (HPLC/PDA) and a Luna C18 column (250 mm x 4.6 mm). The flavans were eluted from the column using an acetonitrile gradient from 10% to 30% ACN over a period of 20 minutes, followed by 60% ACN for five minutes. The results are set forth in Table 4. A profile of the HPLC purification is shown in Figure 2. The flavans were quantified based on retention time and PDA data using catechin and epicatechin as standards. The retention times for the two major flavans were 12.73 minutes and 15.76 minutes, respectively.

**Table 4. Flavan Content in Active Plant Extracts**

| **Active Extracts from Heart woods of *Acacia. catechu*** | **Weight of Extract** | **% Extractible from BioMass** | **% Flavans in Extract** |
|---|---|---|---|
| Aqueous Extract | 10.8 g | 18.0% | 0.998% |
| Organic Extract | 27.2 g | 45.3% | 30.37% |

The flavan content in a standardized extract (Lot #UG0407-050420) isolated from whole plants of *Unicaria gambir* were quantified by HPLC using a PhotoDiode Array detector (HPLC/PDA) and a Luna C18 column (250 mm x 4.6 mm). The flavans were eluted from the column using an acetonitrile gradient from 10% to 30% ACN over a period of 20 minutes, followed by 60% ACN for five minutes. The flavans were quantified based on retention time and PDA data using catechin as standards as 28.6% catechin and 3.9% epicatechin.

### Example 7. Preparation of a Standardized Extract from Acacia catechu

*Acacia catechu* (500 mg of ground bark) was extracted with the following solvent systems. (1) 100% water, (2) 80:20 water:methanol, (3) 60:40 water:methanol, (4) 40:60 water:methanol, (5) 20:80 water:methanol, (6) 100% methanol, (7) 80:20 methanol:THF, (8) 60:40 methanol:THF. The extracts were concentrated and dried under low vacuum. The identification of the chemical components in each extract was achieved by HPLC using a PhotoDiode Array detector (HPLC/PDA) and a 250 mm x 4.6 mm C18 column. The chemical components were quantified based on retention time and PDA data using catechin and epicatechin as standards. The results are set forth in Table 5. As shown in Table 5, the flavan extract generated from solvent extraction with 80% methanol/water provided the best concentration of flavan components.

**Table 5. Solvents for Generating Standardized Flavan Extracts from Acacia catechu**

| **Extraction Solvent** | **' Weight of Extract** | **% Extractible from BioMass** | **Total amount of Catechins** | **% Catechins in Extract** |
|---|---|---|---|---|
| 100% water | 292.8 mg | 58.56% | 13 mg | 12.02% |
| water:methanol | 282.9 mg | 56.58% | 13 mg | 11.19% |
| (80:20) | | | | |
| water:methanol | 287.6 mg | 57.52% | 15 mg | 13.54% |
| (60:40) | | | | |
| water:methanol | 264.8 mg | 52.96% | 19 mg | 13.70% |
| (40:60) | | | | |
| water:methanol | 222.8 mg | 44.56% | 15 mg | 14.83% |
| (20:80) | | | | |
| 100% methanol | 215.0 mg | 43.00% | 15 mg | 12.73% |
| methanol:THF | 264.4 mg | 52.88% | 11 mg | 8.81% |
| (80:20) | | | | |
| methanol:THF | 259.9 mg | 51.98% | 15 mg | 9.05% |
| (60:40) | | | | |

### Example 8. Preparation of Standardized Free-B-Ring Flavonoid Extracts from various Scutellaria species

*Scutellaria orthocalyx* (500 mg of ground root) was extracted twice with 25 mL of the following solvent systems. (1) 100% water, (2) 80:20 water:methanol, (3) 60:40 water:methanol, (4) 40:60 water:methanol, (5) 20:80 water:methanol, (6) 100% methanol, (7) 80:20 methanol:THF, (8) 60:40 methanol:THF. The extracts were combined, concentrated and dried under low vacuum. Identification of chemical components in each extract was performed by HPLC using a PhotoDiode Array detector (HPLC/PDA) and a 250 mm x 4.6 mm C18 column. The chemical components were quantified based on retention time and PDA data using baicalein, baicalin, scutellarein, and wogonin as standards. The results are set forth in Table 6.

**Table 6. Quantification of Free-B-Ring Flavonoids Extracted from Scutellaria orthocalyx**

| **Extraction Solvent** | **Weight of Extract** | **% Extractible from BioMass** | **Total amount of Flavonoids** | **% Flavonoids in Extract** |
|---|---|---|---|---|
| 100% water | 96 mg | 19.2% | 0.02 mg | 0.20% |
| Water:methanol | 138.3 mg | 27.7% | 0.38 mg | 0.38% |
| (80:20) | | | | |
| Water:methanol | 169.5 mg | 33.9% | 0.78 mg | 8.39% |
| (60:40) | | | | |
| Water:methanol | 142.2 mg | 28.4% | 1.14mg | 11.26% |
| (40:60) | | | | |
| Water:methanol | 104.5 mg | 20.9% | 0.94 mg | 7.99% |
| (20:80) | | | | |
| 100% methanol | 57.5 mg | 11.5% | 0.99 mg | 10.42% |
| methanol:THF | 59.6 mg | 11.9% | 0. 89 mg | 8.76% |
| (80:20) | | | | |
| methanol:THF | 58.8 mg | 11.8% | 1.10 mg | 10.71% |
| (60:40) | | | | |

*Scutellaria baicalensis* (1000 mg of ground root) was extracted twice using 50 mL of a mixture of methanol and water as follows: (1) 100% water, (2) 70:30 water:methanol, (3) 50:50 water:methanol, (4) 30:70 water:methanol, (5) 100% methanol. The extracts were combined, concentrated and dried under low vacuum. Identification of the chemical components was performed by HPLC using a PhotoDiode Array detector (HPLC/PDA), and a 250 mm x 4.6 mm C18 column. The chemical components in each extract were quantified based on retention time and PDA data using baicalein, baicalin, scutellarein, and wogonin standards. The results are set forth in Table 7.

**Table 7. Quantification of Free-B-Ring Flavonoids Extracted from Scutellaria baicalensis**

| **Extraction Solvent** | **Weight of Extract** | **% Extractible from BioMass** | **Total amount of Flavonoids** | **% Flavonoids in Extract** |
|---|---|---|---|---|
| 100% water | 277.5 mg | 27.8% | 1 mg | 0.09% |
| Water:methanol | 338.6 mg | 33.9% | 1.19mg | 11.48% |
| (70:30) | | | | |
| Water:methanol | 304.3 mg | 30.4% | 1.99 mg | 18.93% |
| (50:50) | | | | |
| Water:methanol | 293.9 mg | 29.4% | 2.29 mg | 19.61% |
| (30:70) | | | | |
| 100% methanol | 204.2 mg | 20.4% | 2.73 mg | 24.51% |

### Example 9. Preparation of a Formulation with a Standardized Free-B-Ring Flavonoid Extract from the Roots of Scutellaria baicalensis and a Standardized Flavan Extract from the Bark of Acacia catechu

A novel composition of matter, referred to herein as UP736 was formulated using two standardized extracts isolated from *Acacia* and *Scutellaria,* respectively, together with one or more excipients. A general example for preparing such a composition is set forth below. The *Acacia* extract used in this example contained >60% total flavans, as catechin and epicatechin, and the *Scutellaria* extract contained >70% Free-B-Ring flavonoids, which was primarily baicalin. The Scutellaria extract contained other minor amounts of Free-B-Ring flavonoids as set forth in Table 8. One or more excipients is added to the composition of matter. The ratio of flavan and Free-B-Ring flavonoids can be adjusted based on the indications and the specific requirements with respect to the biological activity of the product. The quantity of the excipients can be adjusted based on the actual active content of each ingredient. A blending table for each individual batch of product must be generated based on the product specification and QC results for individual batch of ingredients. Additional amounts of active ingredients in the range of 2-5% are recommended to meet the product specification. Table 8 illustrates a blending table that was generated for one batch of UP736 (Lot#G1702).

*Scutellaria baicalensis* root extract (38.5 kg) (lot # RM052302-01) having a Free-B-Ring flavonoid content of 82.2% (baicalin); *Acacia catechu* bark extract (6.9 kg) (lot # RM052902-01) with total flavan content of 80.4%; and excipient (5.0 kg of Candex) were combined to provide a UP736 formulation (50.4 kg) having a blending ratio of 85:15. Table 8 provides the quantification of the active Free-B-Ring flavonoids and flavans of this specific batch of UP736 (Lot#G1702), determined using the methods provided in Examples 4 and 6.

With reference to Table 8, this specific batch of UP736 contains 86% total active ingredients, including 75.7% Free-B-Ring flavonoids and 10.3% flavans. Two different dosage levels of final product in capsule form were produced from this batch of UP736 (50.0 kg): 125 mg per dose (60 capsules) and 250 mg per dose (60 capsules).

Using the same approach, two other batches of UP736 were prepared using a combination of a standardized Free-B-Ring flavonoid extract from *Scutellaria baicalensis* roots and a standardized flavan extract from *Acacia catechu* bark having a blending ratio of 50:50 and 20:80, respectively.

**Table 8. Free-B-Ring Flavonoid and Flavan Content of UP736**

| **ACTIVE COMPONENTS** | | | **% Content** |
|---|---|---|---|
| **1. Flavonoids** | | | |
| | a. Baicalin | | 62.5% |
| | b. Minor Flavonoids | | |
| | | i. Wogonin-7-glucuronide | 6.7% |
| | | ii. Oroxylin A 7-glucuronide | 2.0% |
| | | iii. Baicalein | 1.5% |
| | | iv. Wogonin | 1.1% |
| | | v. Chrysin-7-glucuronide | 0.8% |
| | | vi. 5-Methyl-wogonin-7-glucuronide | 0.5% |
| | | vii. Scutellarin | 0.3% |
| | | viii Norwogonin | 0.3% |
| | | ix. Chrysin | <0.2% |
| | | x. Oroxylin A | <0.2% |
| | **c. Total Free-B-Ring Flavonoids** | | **75.7%** |
| **2. Flavans** | | | |
| | a. | Catechin | 9.9% |
| | b. | Epicatechin | 0.4% |
| | c. | **Subtotal Flavans** | **10.3%** |
| **3. Total Active Ingredients** | | | **86%** |

### Example 10. Demonstration of the Synergistic Effect of a Composition Comprised of a Mixture of UP736 aud Aspirin on the Inhibition of Arachinodic Acid Induced Platelet Aggregation

The synergistic effect of a composition comprised of a mixture of UP736 and aspirin on the inhibition of arachidonic acid induced platelet aggregation was demonstrated in a platelet aggregation assay using platelet rich plasma prepared from New Zealand Rabbits. The rabbits (2.75 ± 0.25 kg) were treated with trisodium citrate (final concentration of 0.13 M). UP736, aspirin or combinations thereof were dissolved in 0.3% DMSO and incubated with the plasma at 37°C for 5 minutes. Agonist or antagonist effect of the compounds was quantified by optical density of aggregation. Significance criteria used for agonist effect is that ≥50% platelet aggregation relative to arachidonic acid response. Significance criteria used for antagonist is that ≥50% inhibition of arachidonic acid-induced platelet aggregation. In this assay, various concentrations of UP736 (10, 2 and 0.2 µM) and aspirin (30 and 3 µM) were tested individually as either agonist or antagonist ofplatelet aggregation in platelet rich rabbit plasma. Table 9 shows the results of the test (Test #1).

**Table 9. Synergistic Effect of Mixtures of UP736^{®} and Aspirin on Inhibition of AA induced Platelet Aggregation**

| **Aspirin Concentration (µM)** | **UP736 Concentration (µM)** | **N** | **Agonist Effect** | **Antagonist Effect** | |
|---|---|---|---|---|---|
| 0 | 10.0 | 2 | 0% | 3% | |
| 0 | 2.0 | 2 | 0% | 0% | |
| 0 | 0.2 | 2 | 0% | 0% | |
| 3.0 | 10.0 | 2 | 0% | 100% | |
| 3.0 | 2.0 | 2 | 0% | 100% | |
| 3.0 | 0.2 | 2 | 0% | 100% | |
| 30.0 | 0 | 2 | 0% | 100% | |
| 3.0 | 0 | 2 | 0% | 1% | |

In another assay (Test# 2) performed under the same experimental conditions, mixtures of UP736 at lower concentrations (0.2, 0.067, 0.022 and 0.007 µM) and aspirin at a concentration of 3 µM were tested The results are set forth in Table 10.

The results of the platelet aggregation study demonstrate that UP736 alone has little anti-aggregatory activity at concentrations up to 10 µM (Tables 9 and 10). However, the anti-platelet aggregation activity of aspirin at a very low dosage (3 µM) was significantly increased by UP736 even at the lowest concentration tested (0.007µM).

**Table 10. Synergistic Effect of a Mixture of UP736^{®} and Aspirin on Inhibition of AA induced Platelet Aggregation**

| **Aspirin Concentration (µM)** | **UP736 Concentration (µM)** | **N** | **Agonist Effect** | **Antagonist Effect** |
|---|---|---|---|---|
| 0 | 0.2 | 2 | 0% | 9% |
| 3.0 | 0.2 | 2 | 0% | 100% |
| 3.0 | 0.067 | 2 | 0% | 100% |
| 3.0 | 0.022 | 2 | 0% | 100% |
| 3.0 | 0.007 | 2 | 0% | 100% |
| 3.0 | 0 | 2 | 0% | 8% |

### Example 11. Effect of UP736 and Mixtures of UP736 and Aspirin on Bleeding Time

Although UP736 exhibited a synergistic effect with aspirin in the inhibition of platelet aggregation (Tables 9 and 10), neither UP736 alone nor in combination with aspirin showed a substantial effect on bleeding time in mice. The bleeding time assay was conducted according to the method described by Minsker and Kling (1997) Thrombosis Research 10:619-622) and Butler et al. (1982) Thromb Haemostas 47:46-49. Test articles were administered to five ICR male mice one hour before standardized transection of the tip (1.0 mm) of each tail. The mice restrained in holders were immediately suspended vertically with the tail tips immersed in a test tube containing saline at 37°C. There was no maximum cut-off time set. The measurements started as actual bleeding was observed and stopped as bleeding ceased for 15 seconds or longer at any time. Data were analyzed using the Student's t-test. In one assay (Test #3), aspirin alone at 3, 10, 65 and 100 mg/kg or UP736 at a dose of 100 mg/kg, alone or in combination with aspirin at 3, 10 and 65 mg/kg was administered orally to groups of 5 ICR male mice. The results are set forth in Table 11, which shows average bleeding times and percent increases in bleeding time in treatment groups over the vehicle control. With reference to Table 11, the results demonstrate that the effect of UP73 6 on bleeding time is not significant and the effect of UP736 in combination with aspirin at increased concentrations ranging from 3 mg/kg to 65 mg/kg was limited, and was smaller than the effect conferred by 100 mg/kg of aspirin alone. The assay was repeated under the same experimental conditions (Test # 4). The results are set forth in Table 12, which shows average bleeding times and percent increases in bleeding time in treatment groups relative to vehicle control (n=5). As can be seen in Tables 11 and 12, very consistent results were obtained in the two experiments. The data from the two experiments were combined and presented in Table 13 and Figure 3 (n= 9-10).

**Table 11. Average Bleeding Time and % Increase in Bleeding Time in Test #3 (n=5)**

| **Treatment Group** | **Average Bleeding Time ± SD (Sec)** | **% Change Relative to Vehicle Group** |
|---|---|---|
| Vehicle | 65 ± 11.0 | - |
| 3 mg/kg Aspirin | 77.2 ± 13.0 | 18.7 ↑ |
| 10 mg/kg Aspirin | 74.4 ± 15.4 | 14.5 ↑ |
| 65 mg/kg Aspirin | 105 ± 15.1 | 61.5* ↑ |
| 100 mg/kg Aspirin | 119 ± 14.0 | 83* ↑ |
| 3 mg/kg Aspirin + 100 mg/kg UP736 | 82 ± 12.6 | 26.2^{†} ↑ |
| 10 mg/kg Aspirin + 100 mg/kg UP736 | 76.5±17.7 | 17.7^{†}↑ |
| 65 mg/kg Aspirin + 100 mg/kg UP736 | 113.2 ± 33.3 | 74.2^{†}↑ |
| 100 mg/kg UP736 | 76.4 ± 13.0 | 17.5 ↑ |

**Table 12. Average Bleeding Time and % Increase in Bleeding Time in Test #4 (n=5)**

| **Treatment Group** | **Average Bleeding Time ± SD (Sec)** | **% Change Relative to Vehicle Group** |
|---|---|---|
| Vehicle | 72.6 ± 13.0 | - |
| 3 mg/kg Aspirin | 89.4 ± 9.7 | 23.1 ↑ |
| 10 mg/kg Aspirin | 84.6 ± 9.0 | 16.5 ↑ |
| 65 mg/kg Aspirin | 96 ± 6.4 | 32.2* ↑ |
| 100 mg/kg Aspirin | 126.2 ± 14.3 | 73.8* ↑ |
| 3 mg/kg Aspirin + 100 mg/kg UP736 | 92.0 ± 9.3 | 26.7* ↑ |
| 10 mg/kg Aspirin + 100 mg/kg UP736 | 92.6 ± 16.4 | 27.5* ↑ |
| 65 mg/kg Aspirin + 100 mg/kg UP736 | 112.8 ± 16.0 | 55.3 * ↑ |
| 100 mg/kg UP736 | 83.4 ± 6.8 | 14.9 ↑ |

**Table 13. Average Bleeding Time and % Increase in Bleeding Time (Test # 3 and #4) (n=9-10)**

| **Treatment Group** | **Average Bleeding Time ± SD (Sec)** | **% Change Relative to Vehicle Group** | **P-Value** |
|---|---|---|---|
| Vehicle | 68.8 ± 12.0 | - | - |
| 3 mg/kg Aspirin | 83.3 ± 12.6 | 21.7 ↑ | 0.016854 |
| 10 mg/kg Aspirin | 79.5 ± 13.1 | 15.6 ↑ | 0.072734 |
| 65 mg/kg Aspirin | 100.5 ± 11.9 | 46.1 ↑ | 1.32E-05 |
| 100 mg/kg Aspirin | 122.6 ± 13.9 | 78.2 ↑ | 2. 85E-08 |
| 3 mg/kg Aspirin + 100 mg/kg UP736 | 87.0 ± 11.7 | 26.5 t | 0.002958 |
| 10 mg/kg Aspirin + 100 mg/kg UP736 | 85.4 ± 17.9 | 24.1 ↑ | 0.028458 |
| 65 mg/kg Aspirin + 100 mg/kg UP736 | 113.0 ± 25.5 | 64.2 ↑ | 0.00013 |
| 100 mg/kg UP736 | 79.9 ± 10.5 | 16.1 ↑ | 0.041085 |

To verify the bleeding time results, UP736 or UP736 in combination with aspirin was tested again in an independent study (Test #5) using a modified procedure carried out. UP736 was administered orally at a dose of 100 mg/kg, alone or in combination with aspirin at 3, 10 and 30 mg/kg to groups of 5 ICR derived male mice, weighing 22 ± 2 g, 1 hour before transection of the tip (0.3 mm) of each tail. In addition, aspirin alone at 3, 10, 30 and 100 mg/kg was similarly given to mice. The mice, in holders, were immediately suspended vertically with the distal 2 cm of each tail immersed in a test tube containing saline at 37°C. A maximum cut-off time of 180 seconds was set. If bleeding ceased for 15 seconds or longer at any time during the 180 seconds observation period, the measurements were discontinued and any subsequent bleeding would not be considered. Prolongation of bleeding time by 50 percent or more (50%) relative to a control group of animals was considered significant. The results are consistent with those obtained from the above-described experiments (Test #3 and # 4). The effect of UP736 at a dose of 100 mg/kg on bleeding time was not significant and the effect of UP736 at 100 mg/kg in combination with aspirin at 30 mg/kg was smaller than the effect conferred by aspirin alone at 100 mg/kg (Table 14 and Figure 4).

**Table 14. Average Bleeding Time and % Increase in Bleeding Time in Test #5 (n=5)**

| **Treatment Group** | **Average Bleeding Time ± SD (Sec)** | **% Change Relative to Vehicle Group** |
|---|---|---|
| Vehicle | 44.4 ± 15.0 | - |
| 3 mg/kg Aspirin | 42.8 ± 26.2 | 0 |
| 10 mg/kg Aspirin | 39.8 ± 22.4 | 0 |
| 30 mg/kg Aspirin | 56.4 ± 27.3 | 27 ↑ |
| 100 mg/kg Aspirin | 75.0 ± 38.8 | 70* ↑ |
| Vehicle 2 (20% DMSO) | 55.4 ± 32.5 | - |
| 3 mg/kg Aspirin + 100 mg/kg UP736 | 53.2 ± 27.1 | 0 |
| 10 mg/kg Aspirin + 100 mg/kg UP736 | 69.6 ± 45.4 | 27 ↑ |
| 30 mg/kg Aspirin + 100 mg/kg UP736 | 87.2 ± 59.2 | 58 * ↑ |
| 100mg/kg UP736 | 45.4 ± 23.1 | 0 |

In view of the above, it will be appreciated that the invention also encompasses the following items:
1. A composition of matter comprised of a mixture of at least one Free-B-Ring flavonoid, at least one flavan and at least one agent selected from the group consisting of an injectable anticoagulant, an oral anticoagulant, an antiplatelet agent, an anti-angina drug, a non-steroidal anti-inflammatory drug (NSAID) or a cyclooxygenase-2 (COX-2) selective inhibitor.
2. The composition of item 1 wherein the ratio ofFree-B-Ring flavonoid to flavan in said composition is selected from the range of about 99:1 Free-B-ring flavonoid:flavan to about 1:99 of Free-B-Ring flavonoid:flavan.
3. The composition of item 2 wherein the ratio of Free-B-Ring flavonoid:flavan in the composition of matter is about 85:15.
4. The composition of item 1 wherein said Free-B-Ring flavonoid is selected from the group of compounds having the following structure: 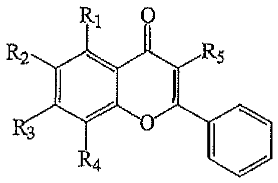 wherein
   R₁, R₂, R₃, R₄, and R₅ are independently selected from the group consisting of -H, - OH, -SH, -OR, -SR, -NH₂, -NHR, -NR₂, -NR₃⁺X⁻, a carbon, oxygen, nitrogen or sulfur, glycoside of a single or a combination ofmultiple sugars including, aldopentoses, methyl-aldopentose, aldohexoses, ketohexose and their chemical derivatives thereof;
   wherein
   R is an alkyl group having between 1-10 carbon atoms; and X is selected from the group ofpharmaceutically acceptable counter anions including, hydroxyl, chloride, iodide, sulfate, phosphate, acetate, fluoride and carbonate.
5. The composition of item 1 wherein said flavan is selected from the group of compounds having the following structure: 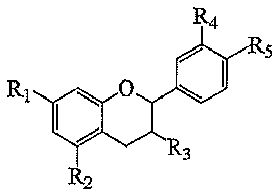 wherein
   R₁, R₂, R₃, R₄ and R₅ are independently selected from the group consisting ofH, -OH, -SH, -OCH₃, -SCH₃, -OR, -SR, -NH₂, -NRH, -NR₂, -NR₃⁺X⁻, esters of substitution groups, independently selected from the group consisting of gallate, acetate, cinnamoyl and hydroxyl-cinnamoyl esters, trihydroxybenzoyl esters and caffeoyl esters; a carbon, oxygen, nitrogen or sulfur glycoside of a single or a combination of multiple sugars including, aldopentoses, methyl aldopentose, aldohexoses, ketohexose and their chemical derivatives thereof dimer, trimer and other polymerized flavans;
   wherein
   R is an alkyl group having between 1-10 carbon atoms ; and X is selected from the group of pharmaceutically acceptable counter anions including, but not limited to hydroxyl, chloride, iodide, sulfate, phosphate, acetate, fluoride, carbonate.
6. The composition of item 1 wherein said Free-B-Ring flavonoid and said flavan are obtained by organic synthesis or are isolated from a plant.
7. The composition of item 6 wherein said Free-B-Ring flavonoid and said flavan are isolated from a plant part selected from the group consisting of stems, stem barks, trunks, trunk barks, twigs, tubers, roots, root barks, young shoots, seeds, rhizomes, flowers and other reproductive organs, leaves and other aerial parts.
8. The composition of item 6 wherein said Free-B-Ring flavonoid is isolated from a plant family selected from the group consisting of *Annonaceae, Asteraceae, Bignoniaceae, Combretaceae, Compositae, Euphorbiaceae, Labiatae, Lauranceae, Leguminosae, Moraceae, Pinaceae, Pteridaceae, Sinopteridaceae, Ulmaceae* and *Zingiberacea.*
9. The composition of item 6 wherein said Free-B-Ring flavonoid is isolated from a plant genus selected from the group consisting of *Desmos, Achyrocline, Oroxylum, Buchenavia, Anaphalis, Cotula, Gnaphalium, Helichrysum, Centaurea, Eupatorium, Baccharis, Sapium, Scutellaria, Molsa, Colebrookea, Stachys, Origanum, Ziziphora, Lindera, Actinodaphne, Acacia, Derris, Glycyrrhiza, Millettia, Pongamia, Tephrosia, Artocarpus, Ficus, Pityrogramma, Notholaena, Pinus, Ulmus* and *Alpinia.*
10. The composition item 6 wherein said flavan is are isolated from a plant species selected from the group consisting of the *Acacia catechu, Acacia concinna, Acacia farnesiana, Acacia Senegal, Acacia speciosa, Acacia arabica, Acacia caesia, Acacia pennata, Acacia sinuate, Acacia mearnsii, Acacia picnantha, Acacia dealbata, Acacia auriculiformis, Acacia holoserecia* and *Acacia mangium; Uncaria gambir, Uncaria lanosa, Uncaria hirsute, Uncaria africana, Uncaria elliptica, Uncaria orientalis, Uncaria attenuate, Uncaria acida, Uncaria homomalla, Uncaria sessilifructus, Uncaria sterrophylla, Uncaria bernaysii, Uncaria sinensis, Uncaria callophylla, Uncaria rhychophylla, Uncaria tomentosa, Uncaria longiflora, Uncaria hirsute, Uncaria cordata,* and *Uncaria borneensis.*
11. The composition of item 6 wherein said Free-B-Ring flavonoid is isolated from a plant or plants in the *Scutellaria* genus of plants and said flavan is isolated from a plant or plants in the *Acacia* and *Uncaria* genus of plants.
12. The composition of item 1 wherein said injectable anticoagulant is selected from the group consisting of heparin, dalteparin, enoxaparin and tinzaparin.
13. The composition of item 1 wherein said oral anticoagulant is selected from the group consisting of warfarin, vitamin K antagonists and vitamin K reductase inhibitors.
14. The composition of item 1 wherein said antiplatelet agent is selected from the group consisting of aspirin, clodipogrel and dipyridamole.
15. The composition of item 1 wherein said anti-angina drug is selected from the group consisting of nitrates, beta-blockers, calcium blockers, angiotensin-converting enzyme inhibitors, and potassium channel activators.
16. The composition of item 1 wherein said NSAID is selected from the group consisting of acetaminophen, ibuprofen, naproxen, diclofenac, salicylates and indometacin.
17. The composition of item 1 wherein said COX-2 selective inhibitor is selected from the group consisting of rofecoxib, celecoxib, etodolac and meloxicam.
18. A method for and preventing and treating diseases and conditions related to platelet aggregation and platelet-induced thrombosis said method said method comprising administering to a host in need thereof an effective amount of a composition comprising a mixture of Free-B-Ring flavonoids and flavans together with a pharmaceutically acceptable carrier.
19. The method of *item* 18 wherein the composition is administered in a dosage selected from 0.01 to 200 mg/kg of body weight.
20. The method of item 18 wherein the routes of the administration are selected from the group consisting of oral, topical, suppository, intravenous, and intradermic, intragaster, intramusclar, intraperitoneal and intravenous administration.
21. The method of item 18 wherein said diseases and conditions related to platelet aggregation and platelet-induced thrombosis are selected from the group consisting of deep vein thrombosis, pulmonary embolism, atherosclerosis, myocardial infarction, thrombosis in cerebral vessels and/or embolism of cerebral vessels leading to cerebrovascular events, thrombosis or peripheral circulation and/or microcirculation resulting in ischemia and infarction, atrial fibrillation that is associated with the stasis of blood and formation of thrombosis in the left atria, thrombogenic sites including artificial implantations such as mechanical heart valves, defibricators, surgical implantations for drug delivery, and artificial hips, joints and other exogenous organs.
22. A method of using a composition of matter comprised of a mixture of at least one Free-B-Ring flavonoid and at least one flavan as an adjuvant and/or a synergistic, and/or a potentiating agent for the delivery of an agent selected from the group consisting of an injectable anticoagulant, an oral anticoagulant, an antiplatelet agent, an anti-angina drug, a non-steroidal anti-inflammatory drug (NSAID) or a cyclooxygenase-2 (COX-2) selective
   inhibitor, comprising administration of said agent to a host in need thereof in combination with said mixture of Free-B-Ring flavonoid and flavan.
23. The method of item 22 wherein said injectable anticoagulant is selected from the group consisting ofheparin, dalteparin, enoxaparin and tinzaparin.
24. The method of item 22 wherein said oral anticoagulant is selected from the group consisting of warfarin, vitamin K antagonists and vitamin K reductase inhibitors.
25. The method of item 22 wherein said antiplatelet agent is selected from the group consisting of aspirin, clodipogrel and dipyridamole.
26. The method of item 22 wherein said anti-angina drug is selected from the group consisting of nitrates, beta-blockers, calcium blockers, angiotensin-converting enzyme inhibitors, and potassium channel activators.
27. The method of item 22 wherein said NSAID is selected from the group consisting of acetaminophen, ibuprofen, naproxen, diclofenac, salicylates and indometacin.
28. The method of item 22 wherein said COX-2 selective inhibitor is selected from the group consisting of rofecoxib, celecoxib, etodolac and meloxicam.
29. The method of item 22 wherein the composition is administered in a dosage selected from 0.01 to 200 mg/kg of body weight.
30. The method of item 22 wherein the routes of the administration are selected from the group consisting of oral, topical, suppository, intravenous, and intradermic, intragaster, intramusclar, intraperitoneal and intravenous administration.
31. A method for reducing the standard dosage of an agent selected from the group consisting of an anti-platelet, anti-coagulant, a prophylaxis agent, an NSAID and a COX-2 selective inhibitor said method comprising administration of a composition of matter comprised of a mixture of at least one Free-B-Ring flavonoid and at least one flavan in combination with said anti-platelet, anti-coagulant, prophylaxis agent or NSAID.
32. The method of item 31 wherein the composition is administered in a dosage selected from 0.01 to 200 mg/kg of body weight.
33. The method of item 31 wherein the routes of the administration are selected from the group consisting of oral, topical, suppository, intravenous, and intradermic, intragaster, intramusclar, intraperitoneal and intravenous administration.
34. A method for decreasing or eliminating the side effects caused by acute or chronic administration of an agent selected from the group consisting of an anti-platelet, anti-coagulant, a prophylaxis agent and an NSAID said method comprising administration of a composition of matter comprised of a mixture of at least one Free-B-Ring flavonoid and at least one flavan in combination with said anti-platelet, anti-coagulant, prophylaxis agent, NSAID or COX-2 selective inhibitor.
35. The method of item 34 wherein the composition is administered in a dosage selected from 0.01 to 200 mg/kg of body weight.
36. The method of item 34 wherein the routes of the administration are selected from the group consisting of oral, topical, suppository, intravenous, and intradermic, intragaster, intramusclar, intraperitoneal and intravenous administration.
37. A method for counteracting or antagonizing the risks of acute or chronic administration of an agent selected from the group consisting of an anti-platelet, anti-coagulant, a prophylaxis agent, an NSAID and a COX-2 selective inhibitor said method comprising administration of a composition of matter comprised of a mixture of at least one Free-B-Ring flavonoid and at least one flavan in combination with said anti-platelet, anti-coagulant, prophylaxis agent or NSAID.
38. The method of item 37 wherein the composition is administered in a dosage selected from 0.01 to 200 mg/kg of body weight.
39. The method of item 37 wherein the routes of the administration are selected from the group consisting of oral, topical, suppository, intravenous, and intradermic, intragaster, intramusclar, intraperitoneal and intravenous administration.

## Claims

1. A mixture of at least one Free-B-Ring flavonoid and at least one flavan for use as an adjuvant agent, a synergistic agent, a potentiating agent, or combinations thereof for the delivery of an agent selected from the group consisting of an injectable anticoagulant, an oral anticoagulant, an antiplatelet agent, an anti-angina drug, a non-steroidal anti-inflammatory drug (NSAID) or a cyclooxygenase-2 (COX-2) selective inhibitor.

2. The mixture of claim 1, wherein said mixture is a mixture of Free-B-Ring flavonoids and flavans.

3. The mixture of claim 1, wherein said injectable anticoagulant is selected from the group consisting of heparin, dalteparin, enoxaparin and tinzaparin.

4. The mixture of claim 1, wherein said oral anticoagulant is selected from the group consisting of warfarin, vitamin K antagonists and vitamin K reductase inhibitors.

5. The mixture of claim 1, wherein said antiplatelet agent is selected from the group consisting of aspirin, clodipogrel and dipyridamole.

6. The mixture of claim 1, wherein said anti-angina drug is selected from the group consisting of nitrates, beta-blockers, calcium blockers, angiotensin-converting enzyme inhibitors, and potassium channel activators.

7. The mixture of claim 1, wherein said NSAID is selected from the group consisting of acetaminophen, ibuprofen, naproxen, diclofenac, salicylates and indometacin.

8. The mixture of claim 1, wherein said COX-2 selective inhibitor is selected from the group consisting of rofecoxib, celecoxib, etodolac and meloxicam.

9. A mixture of at least one Free-B-Ring flavonoid and at least one flavan in combination with an anti-platelet agent, anti-coagulant agent, prophylaxis agent, NSAID or COX-2 selective inhibitor for use in reducing the standard dosage of the anti-platelet agent, anti-coagulant agent, prophylaxis agent, NSAID or COX-2 selective inhibitor.

10. A mixture of at least one Free-B-Ring flavonoid and at least one flavan in combination with an anti-platelet agent, anti-coagulant agent, prophylaxis agent, NSAID or COX-2 selective inhibitor for use in decreasing or eliminating the side effects caused by acute or chronic administration of the anti-platelet agent, anti-coagulant agent, prophylaxis agent, NSAID or COX-2 selective inhibitor.

11. A mixture of at least one Free-B-Ring flavonoid and at least one flavan in combination with an anti-platelet agent, anti-coagulant agent, prophylaxis agent agent, NSAID or COX-2 selective inhibitor for use in antagonizing the risks of acute or chronic administration of the anti-platelet agent, anti-coagulant agent, prophylaxis agent, NSAID or COX-2 selective inhibitor.

12. A composition comprising a therapeutically effective amount of a mixture of Free-B-Ring flavonoids and flavans and a pharmaceutically acceptable carrier for use in preventing or treating diseases or conditions related to platelet aggregation or platelet-induced thrombosis.

13. The composition of claim 12, for administration in a dosage selected from 0.01 to 200 mg/kg of body weight.

14. The composition of claim 12, wherein the composition is formulated for oral, topical, suppository, intravenous, and intradermic, intragaster, intramusclar, intraperitoneal or intravenous administration.

15. The composition of claim 12, wherein said diseases and conditions related to platelet aggregation or platelet-induced thrombosis are selected from the group consisting of deep vein thrombosis, pulmonary embolism, atherosclerosis, myocardial infarction, thrombosis in cerebral vessels and/or embolism of cerebral vessels leading to cerebrovascular events, thrombosis or peripheral circulation and/or microcirculation resulting in ischemia and infarction, atrial fibrillation that is associated with the stasis of blood and formation of thrombosis in the left atria, thrombogenic sites including artificial implantations such as mechanical heart valves, defibricators, surgical implantations for drug delivery, and artificial hips, joints and other exogenous organs.
